# EUROPEAN PATENT SPECIFICATION

(11) **EP 0 616 613 B1**
(45) Date of publication and mention of the grant of the patent: **17.03.1999**
(21) Application number: 92924777.3
(22) Date of filing: 03.12.1992
(51) Int. Cl.: C07K 7/06, C07K 7/08, C07K 14/00, A61K 38/00, A61K 39/00, G01N 33/68

(54) **FRAGMENTS OF PRION PROTEINS**
Fragmente von Prion Proteinen.
FRAGMENTS DE PROTEINES DE PRION

(30) Priority: 03.12.1991 GB 9125747; 10.07.1992 GB 9214663
(43) Date of publication of application: 28.09.1994
(73) Proprietor: PROTEUS MOLECULAR DESIGN LIMITED, Macclesfield, Cheshire SK11 0JL (GB)
(72) Inventor: FISHLEIGH, Robert, Vincent Bradley Smithy Cottage, Cheshire SK11 0HD (GB); ROBSON, Barry The Old Bakery 22A Town Street, Cheshire SK6 5AA (GB); MEE, Roger, Paul, Manchester M20 9DZ (GB)
(74) Representative: Grant, Anne Rosemary
(86) International application number: PCT/GB92/02246
(87) International publication number: WO 93/11155

(56) References cited:
- CELL vol. 46, 1 August 1986, CAMBRIDGE, NA US pages 417 - 428 BASLER ET AL. 'Scrapie and Cellular PrP Isoforms Are Encoded by the Same Chromosomal Gene'
- PROCEEDINGS OF THE NATIONAL ACADEMY OF SCIENCES OF USA vol. 87, no. 7, April 1990, WASHINGTON US pages 2476 - 2480 W. GOLDMANN ET AL. 'Two alleles of a neural protein gene linked to scrapie in sheep'
- JOURNAL OF IMMUNOLOGY vol. 147, no. 10, 15 November 1991, BALTIMORE US pages 3568 - 3574 M. ROGERS ET AL. 'EPITOPE MAPPING OF THE SYRIAN HAMSTER PRION PROTEIN UTILIZING CHIMERIC AND MUTANT GENES IN A VACCINIA VIRUS EXPRESSION SYSTEM'
- JOURNAL OF MOLECULAR RECOGNITION vol. 4, no. 2/3, June 1991, pages 85 - 91 A.D. MARTINO 'Production and Characterization of Antibodies to Mouse Scrapie-Amyloid Protein Elicited by Non-carrier Linked Synthetic Peptide Immunogens'
- JOURNAL OF VIROLOGY vol. 65, no. 7, July 1991, pages 3667 - 3675 D.C. BOLTON ET AL. 'Molecular Location of a Species-Specific Epitope an the Hamster Scrapie Agent Protein'
- NEUROLOGY vol. 40, no. 3, March 1990, pages 513 - 517 J. SAFAR ET AL. 'Scrapie-associated precursor proteins'

## Description

The present invention relates to synthetic polypeptides. In particular it relates to synthetic polypeptides which emulate the three-dimensional structures and/or electrostatic surfaces and/or other physical, chemical and structural properties of specific regions of proteins thought to be the involved in the molecular pathology of spongiform encephalopathies. It is of particular interest to the design of immunodiagnostics, vaccines and other medical, veterinary or scientific agents in relation to human, bovine and ovine spongiform encephalopathies.

Spongiform encephalopathies are a group of degenerative neurological diseases. Examples have been found in a number of species including sheep (where it is known as scrapie), cows (BSE) and humans (Creutzfeldt-Jakob disease (CJD) and kuru) (Review article, Taylor, D.M. Veterinary Record 125, 413-415 (1989)). Similar con itions have also been found in the wild mink population and in captive kudus (a kind of antelope) and tigers. It has been variously reported that BSE can be transmitted under laboratory conditions to mice and pigs. This crossing of species barriers by the infective agent has led to increased concern that transfer to humans could occur.

These diseases are characterised by a slow incubation time of four to five years after which the clinical symptoms of progressive degeneration of mental state, including aggressiveness and lack of coordination, appear. Post mortems reveal a characteristic pattern of vacuolation in brain tissue due to the destruction of neural cells, and the deposition of unusual protein fibres.

Although the form of the disease found in sheep (scrapie) has been known for many years, spongiform encephalopathies have come to prominence within the last decade following the appearance of BSE in cattle farms. The incidence of BSE in the United Kingdom has increased markedly during this period and public concern over the possible transmission of the disease to humans has led to a collapse in the beef market. Thus for both veterinary and economic reasons, there is an urgent need for diagnostic agents to detect infection and for vaccines to prevent infection.

It is believed that the causative agent of scrapie and its counterparts in other animals is a so-called "prion", that is an infective particle comprising protein only and no nucleic acid, the presence of the latter being required in the case of a conventional virus. In scrapie, one particular protein (termed prion protein, PrP^{SC}) has been found to co-purify with infectivity and can produce a scrapie-like condition in brain cell cultures from other animals, such as hamsters, under laboratory conditions. PrP^{SC} is the only known component of the characteristic protein fibres deposited in the brain tissue of scrapie-infected sheep. The term "PrP^{SC}" as used herein should be taken to refer not only to the specific Prion protein identified in sheep but also to those homologous proteins found in many other species which appear to undergo a structural modification as described hereinafter. The term "PrP^{C}" shall be used in respect of the normal cellular counterpart to PrP^{SC}.

The major problem in the search for a specific diagnostic agent or synthetic vaccine against the scrapie agent PrP^{SC} is that it is almost identical to the natural form of the protein PrP^{C}. The natural function of this protein is not yet understood but the remarkably strong conservation of primary structure between homologous proteins from different species suggests that it has an essential structural or functional role within the organism.

In spite of the almost identical form of these prions to the natural proteins, we have deduced synthetic peptide structures comprising at least one antigenic property, such as an epitopic site and these synthetic peptides may be used to produce diagnostic agents and vaccines.

The responses of the B and T cells of the immune system are not specified by a global recognition of a whole protein but rather by recognition of a small region of the protein surface known as epitopic site. Such sites may be formed by a continuous section of peptide chain or may be discontinuous, where separated sections of peptide chain are brought together at the protein surface due to folding of the chain. One aim in producing a synthetic peptide vaccine is to mimic the structure of a particular epitope and thereby cause a primary immune response leading to the production of memory B cells which will secrete antibodies on subsequent exposure to the parent protein so producing a greatly enhanced response to secondary infection. A similar mechanism via priming of the cytotoxic T cells to respond more vigorously to a particular antigen will also occur.

However, problems exist with the application of traditional methods of vaccine production to this disease as it is believed that the molecular structure of the protein prion rather than nucleic acid sequence passes on infectivity in the prion. The usual method of viral vaccine production involves the inactivation of the virus in some way to destroy infectivity whilst preserving epitopic sites. Such techniques as heat treatment or serial passaging of the virus through a culture are used, but these approaches would not lead to a loss of infectivity of a prion unless conditions were such as to cause protein denaturation. If the conditions are severe enough to inactivate the prion protein then denaturation of the protein occurs and any epitopic sites are lost. Thus there is a major problem in trying to obtain antigenic but non-infective prion proteins by conventional routes. It is known, for example, that the scrapie agent in sheep is particularly resistant to chemical or physical inactivation (Hodgson, J. Bio/Technology 8 990 (1990)).

Our invention relates to certain synthetic polypeptides having at least one antigenic site of a prion protein. Preferably the prion protein is of a form which only exists in nervous tissue of a mammal suffering from spongiform encephalopathy.

We have found that prion proteins of the type mentioned above comprise six regions of interest, labelled A to F, and two related frame shift peptide sequences, viz:1) a repeating section in region E having undergone a nucleic acid coding sequence frame shift of +1 (FSa) and 2) the repeating section in region E having undergone a nucleic acid coding sequence frame shift of -1 (FSb).

With regard to region A, our invention provides a synthetic peptide sequence according to general formula (I) : 
X-(R1-Lys-His-R2)-Ala-Gly-Ala-Ala-Ala-R3-Gly-Ala-Val-
Val-Gly-Gly-Leu-Gly-Gly-Tyr-Met-Leu-Gly-Ser-Ala-Met-
Ser-(Arg-Pro-R4-R5)-Y
(I)
 wherein
R₁ is an amino acid residue selected from Met, Leu and Phe;
R₂ is either Met or Val;
R₃ is Ala or is absent;
R₄ and R₅ are independently an amino acid residue selected from Leu, Ile and Met; one or more residues within brackets maybe present or absent with the proviso that if they are present they are attached to the rest of the peptide in sequence; and X and Y may each independently be absent or independently be one or more additional amino acid residues.

It will be apparent for example that the residues at the N-terminal of the sequence may be present as "R₂"-or "His-R₂-," or "Lys-His-R₂-" or "R₁-Lys-His-R₂-." Similarly, the preferable residues at the C-terminal may be present as "-Arg", or "-Arg-Pro," or "-Arg-Pro-R₄," or "-Arg-Pro-R₄-R₅."

Preferably, R₁, if present, is Met, R₃ is Ala and R₅, if present, is Ile. Also, if R₂ is Met then R₄, if present, is Ile. Below are preferred sequences (Seq. I.D. No: 1 and Seq. I.D. No: 2) of formula I relating to bovine and ovine and to human prion proteins respectively: 
Seq. I.D. No: 1
X-(Met-Lys-His-Val)-Ala-Gly-Ala-Ala-Ala-Ala-Gly-Ala-
Val-Val-Gly-Gly-Leu-Gly-Gly-Tyr-Met-Leu-Gly-Ser-Ala-
Met-Ser-(Arg-Pro-Leu-Ile)-Y;
 and

Seq. I.D. No: 2
X-(Met-Lys-His-Met)-Ala-Gly-Ala-Ala-Ala-Ala-Gly-Ala-
Val-Val-Gly-Gly-Leu-Gly-Gly-Tyr-Met-Leu-Gly-Ser-Ala-
Met-Ser-(Arg-Pro-Ile-Ile)-Y.

A particularly preferred sequence according to formula I is Seq. I.D. No: 51

Lys-His-Met-Ala-Gly-Ala-Ala-Ala-Ala-Gly-Ala-
Val-Val-Gly-Gly-Leu-Gly-Gly-Tyr-Met-Leu-Gly-Ser-Ala-
Met-Ser-Arg-Gly-Cys.

Naturally, our invention encompasses significant sub-fragments of the sequence according to formula I above and preferred sub-fragments are: i) X-(His-R2-Ala-Gly)-Ala-Ala-Ala-R3-Gly-Ala-Val- Val-(Gly-Gly-Leu-Gly)-Y and ; 
ii) X-(Gly-Gly-Leu-Gly)-Gly-Tyr-Met-Leu-Gly-Ser-
Ala-Met-Ser-(Arg-Pro-R4-R5)-Y
 wherein R₂, R₃, R₄, R₅, X and Y are as defined for formula I and one or more residues in brackets may be absent or present as in formula I.

It will be clear from the foregoing that preferred sub-fragments relating to both bovines and ovines are 
Seq. I.D. No: 3
i) X-(His-Val-Ala-Gly)-Ala-Ala-Ala-Ala-Gly-Ala-
Val-Val-Gly-(Gly-Leu-Gly-Gly)-Y;
 and 
Seq. I.D. No: 4
ii) X-(Gly-Gly-Leu-Gly)-Gly-Tyr-Met-Leu-Gly-Ser-
Ala-Met-Ser-(Arg-Pro-Leu-Ile)-Y.
 Similarly, preferred sub-fragments for humans are: 
Seq. I.D. No: 5
i) X-(His-Met-Ala-Gly)-Ala-Ala-Ala-Ala-Gly-Ala-
Val-Val-Gly-(Gly-Leu-Gly-Gly)-Y;
 and 
Seq. I.D. No: 6
ii) X-(Gly-Gly-Leu-Gly)-Gly-Tyr-Met-Leu-Gly-Ser-
Ala-Met-Ser-(Arg-Pro-Ile-Ile)-Y.
 With regard to region B, our invention provides a synthetic peptide sequence according to general formula II: 
X-(Ser-Ala-Met-Ser)-Arg-Pro-R4-R5-His-Phe-Gly-R6-
Asp-R7-Glu-Asp-Arg-Tyr-Tyr-Arg-Glu-Asn-Met-R8-Arg-
(Tyr-Pro-Asn-Gln)-Y
(II)
 wherein
R₄ and R₅ are the same as in formula I;
R₆ is either Asn or Ser;
R₇ is either Tyr or Trp;
R₈ is an amino acid residue selected from His, Tyr and Asn;
one or more residues within brackets maybe present or absent with the proviso that if they are present they are attached to the rest of the peptide in sequence; and X and Y may each independently be absent or independently be one or more additional amino acid residues.

Preferably in a sequence according to formula II, R₅ is Ile, R₇ is Tyr and R₈ is His or Tyr. Below are preferred sequences of formula II relating to bovine, ovine and human prion proteins respectively: 
Seq. I.D. No: 7
-X-(Ser-Ala-Met-Ser)-Arg-Pro-Leu-Ile-His-Phe-Gly-Ser-
Asp-Tyr-Glu-Asp-Arg-Tyr-Tyr-Arg-Glu-Asn-Met-His-Arg-
(Tyr-Pro-Asn-Gln)-Y;
 
Seq. I.D. No: 8
X-(Ser-Ala-Met-Ser)-Arg-Pro-Leu-Ile-His-Phe-Gly-Asn-
Asp-Tyr-Glu-Asp-Arg-Tyr-Tyr-Arg-Glu-Asn-Met-Tyr-Arg-
(Tyr-Pro-Asn-Gln)-Y;
 and

Seq. I.D. No: 9
X-(Ser-Ala-Met-Ser)-Arg-Pro-Ile-Ile-His-Phe-Gly-Ser-
Asp-Tyr-Glu-Asp-Arg-Tyr-Tyr-Arg-Glu-Asn-Met-His-Arg-
(Tyr-Pro-Asn-Gln)-Y.

Particularly preferred sequences are selected from Seq. I.D. No: 42 
Ser-Ala-Met-Ser-Arg-Pro-Leu-Ile-His-Phe-Gly-
Asn-Asp-Tyr-Glu-Asp-Arg-Tyr-Tyr-Gly-Cys;
 and

Seq. I.D. No: 43
Ser-Ala-Met-Ser-Arg-Pro-Leu-Ile-His-Phe-Gly-
Ser-Asp-Tyr-Glu-Asp-Arg-Tyr-Tyr-Gly-Cys.

Again it will be apparent that our invention encompasses significant sub-fragments of the sequence according to Formula II and a preferred general sub-fragment has the sequence:- 
X-(Ser-Ala-Met-Ser)-Arg-Pro-R4-R5-His-Phe-Gly-R6-
Asp-R7-Glu-Asp-Arg-Tyr-Tyr-(Arg-Glu-Asn-Met)-Y
 wherein R₄ to R₇, X and Y are as defined in formula II and one or more residues in brackets may be present or absent. Preferably, R₅ is Ile and R₇ is Tyr. It will be appreciated that preferred sub-fragments relating to bovines, ovines and humans are respectively; 
Seq. I.D. No: 10
X-(Ser-Ala-Met-Ser)-Arg-Pro-Leu-Ile-His-Phe-Gly-Ser-
Asp-Tyr-Glu-Asp-Arg-Tyr-Tyr-(Arg-Glu-Asn-Met)-Y;
 
Seq. I.D. No: 11
X-(Ser-Ala-Met-Ser)-Arg-Pro-Leu-Ile-His-Phe-Gly-Asn-
Asp-Tyr-Glu-Asp-Arg-Tyr-Tyr-(Arg-Glu-Asn-Met)-Y;
 and

Seq. I.D. No: 12
X-(Ser-Ala-Met-Ser)-Arg-Pro-Ile-Ile-His-Phe-Gly-Ser-
Asp-Tyr-Glu-Asp-Arg-Tyr-Tyr-(Arg-Glu-Asn-Met)-Y.

Our invention provides in respect of region C a synthetic peptide sequence according to general formula III: 
X-(Asn-Met-R8-Arg)-Tyr-Pro-Asn-Gln-Val-Tyr-Tyr-Arg-
Pro-R9-Asp-R10-Tyr-R11-Asn-Gln-Asn-Asn-Phe-Val-His-
(Asp-Cys-Val-Asn)-Y
(III)
 wherein
R₈ is an amino acid residue selected from His, Tyr and Asn;
R₉ is Val or Met;
R₁₀ is an amino acid residue selected from Gln, Glu and Arg;
R₁₁ is Ser or Asn; one or more residues within brackets maybe present or absent with the proviso that if they are present they are attached to the rest of the peptide in sequence and X and Y may each independently be absent or independently be one or more additional amino acid residues.

Preferably in a sequence according to formula III, R₈ is His or Tyr and R₁₁ is Ser. Below are preferred sequences of formula III relating to bovine, ovine and human prion proteins respectively: 
Seq. I.D. No: 13
X-(Asn-Met-His-Arg)-Tyr-Pro-Asn-Gln-Val-Tyr-Tyr-Arg-
Pro-Val-Asp-Gln-Tyr-Ser-Asn-Gln-Asn-Asn-Phe-Val-His-
(Asp-Cys-Val-Asn)-Y;
 
Seq. I.D. No: 14
X-(Asn-Met-Tyr-Arg)-Tyr-Pro-Asn-Gln-Val-Tyr-Tyr-Arg-
Pro-Val-Asp-Arg-Tyr-Ser-Asn-Gln-Asn-Asn-Phe-Val-His-
(Asp-Cys-Val-Asn)-Y;
 and

Seq. I.D. No: 15
X-(Asn-Met-His-Arg)-Tyr-Pro-Asn-Gln-Val-Tyr-Tyr-Arg-
Pro-Met-Asp-Glu-Tyr-Ser-Asn-Gln-Asn-Asn-Phe-Val-His-
(Asp-Cys-Val-Asn)-Y.

Particularly preferred sequences are selected from Seq. I.D. No: 44 
Asn-Met-Tyr-Arg-Tyr-Pro-Asn-Gln-Val-Tyr-Tyr-Arg-Pro-Val-
Asp-Arg-Tyr-Ser-Asn-Gln-Asn-Asn-Phe-Val-His-Gly-Cys;
 and

Seq. I.D. No: 45
Asn-Met-His-Arg-Tyr-Pro-Asn-Gln-Val-Tyr-Tyr-Arg-Pro-Val-
Asp-Gln-Tyr-Ser-Asn-Gln-Asn-Asn-Phe-Val-His-Gly-Cys.

Significant sub-fragments of the sequence according to formula III form part of this invention and a preferred sub-fragment has the sequence:

X-(Arg-Tyr-Pro-Asn)-Gln-Val-Tyr-Tyr-Arg-Pro-R9-Asp-
R10-Tyr-R11-Asn-Gln-Asn-Asn-Phe-Val-His-
(Asp-Cys-Val-Asn)-Y.

Preferred sub-fragments relating to bovines, ovines and humans are respectively: 
Seq. I.D. No: 16
X-(Arg-Tyr-Pro-Asn)-Gln-Val-Tyr-Tyr-Arg-Pro-Val-Asp-
Gln-Tyr-Ser-Asn-Gln-Asn-Asn-Phe-Val-His-
(Asp-Cys-Val-Asn)-Y;
 
Seq. I.D. No: 17
X-(Arg-Tyr-Pro-Asn)-Gln-Val-Tyr-Tyr-Arg-Pro-Val-Asp-
Arg-Tyr-Ser-Asn-Gln-Asn-Asn-Phe-Val-His-
(Asp-Cys-Val-Asn)-Y;
 and

Seq. I.D. No: 18
X-(Arg-Tyr-Pro-Asn)-Gln-Val-Tyr-Tyr-Arg-Pro-Met-Asp-
Glu-Tyr-Ser-Asn-Gln-Asn-Asn-Phe-Val-His-
(Asp-Cys-Val-Asn)-Y.

In respect of region D, our invention provides a synthetic peptide sequence according to general formula IV: 
X-(Tyr-Tyr-R12-R13-Arg)-R14-R15-Ser-R16-R17-R18-Leu-Phe-Ser-
Ser-Pro-Pro-Val-Ile-Leu-Leu-Ile-Ser-Phe-Leu-Ile-Phe-
 
Leu-R19-Val-Gly-Y
(IV)
 wherein
R₁₂ is Asp or Gln;
R₁₃ is Gly or absent;
R₁₄ is Gly or Arg;
R₁₅ is Ala or Ser;
R₁₆ is Ser or absent;
R₁₇ is an amino acid residue selected from Ala, Thr, Met and Val;
R₁₈ is Val or Ile;
R₁₉ is Ile or Met; one or more residues within brackets may be present or absent with the proviso that if they are present they are attached to the rest of the peptide in sequence and X and Y may each independently be absent or independently be one or more additional amino acid residues.

Preferably in a sequence according to formula IV R₁₂ is Gln, R₁₃ is absent, R₁₄ is Gly, R₁₆ is absent, R₁₇ is Val or Met and R₁₉ is Ile.

Preferred sequences of formula IV relating to bovine and ovine and to human prion proteins respectively are given below: 
Seq. I.D. No: 19
X-(Tyr-Tyr-Gln-Arg)-Gly-Ala-Ser-Val-Ile-Leu-Phe-Ser-
Ser-Pro-Pro-Val-Ile-Leu-Leu-Ile-Ser-Phe-Leu-Ile-Phe-
Leu-Ile-Val-Gly-Y;
 and

Seq. I.D. No: 20
X-(Tyr-Tyr-Gln-Arg)-Gly-Ser-Ser-Met-Val-Leu-Phe-Ser-Ser-
Pro-Pro-Val-Ile-Leu-Leu-Ile-Ser-Phe-Leu-Ile-
Phe-Leu-Ile-Val-Gly-Y.

Clearly, it will be recognised that the present invention includes with its ambit significant sub-fragments of the sequence according to formula IV and a preferred general sub-fragment has the sequence:

X-(R14-R15-Ser-R16-R17)-R18-Leu-Phe-Ser-Ser-Pro-Pro-Val-

Ile-(Leu-Leu-Ile-Ser)-Y

Wherein R₁₄ to R₁₈, X and Y are as defined in formula IV and one or more residues within brackets may be present or absent as in formula IV.

It is preferred that in a sub-fragment as given above, R₁₄ is Gly, R₁₆ is absent and R₁₇ is Val or Met. Below are preferred sub-fragments relating to bovines and ovines and to humans respectively: 
Seq. I.D. No: 21
X-(Gly-Ala-Ser-Val)-Ile-Leu-Phe-Ser-Ser-Pro-Pro-Val-
Ile-(Leu-Leu-Ile-Ser)-Y;
 and

Seq. I.D. No: 22
X-(Gly-Ser-Ser-Met)-Val-Leu-Phe-Ser-Ser-Pro-Pro-Val-
Ile-(Leu-Leu-Ile-Ser)-Y.

Our invention provides in respect of Region E three synthetic polypeptide sequences according to general formulae Va, Vb and Vc: 
X-(Pro-Gly-Gly-R20)-Trp-Asn-Thr-Gly-Gly-Ser-Arg-Tyr-
Pro-Gly-Gln-Gly-Ser-Pro-Gly-Gly-Asn-Arg-Tyr-Pro-Pro-
Gln-Gly-(Gly-R21-R22-Trp)-Y     (Va);
 
X-(Gly-Gly-R21-R22-Trp)-Gly-Gln-Pro-His-Gly-Gly-Gly-
R23-Trp-(Gly-Gln-Pro-His)-Y (Vb);
 and

X-(Gly-Gly-Gly-Trp)-Gly-Gln-Gly-Gly-R24-R25-His-R26-
Gln-Trp-Asn-Lys-Pro-R27-Lys-Pro-Lys-Thr-R28-R29-Lys
(-His-R30-Ala-Gly)-Y     (Vc)

Wherein R₂₀, R₂₁, R₂₃ and R₂₄ are each independently either Gly or absent;
R₂₂ is either Gly or Thr;
R₂₅ is either Thr or Ser;
R₂₆ is an amino acid residue selected from Gly, Ser and Asn;
R₂₇ and R₂₈ are each independently either Asn or Ser;
R₂₉ is an amino acid residue selected from Met, Leu and Phe;
R₃₀ is either Val or Met; one or more residues within brackets maybe present or absent with the proviso that if they are present they are attached to the rest of the peptide in sequence; and X and Y may each independently be absent or independently be one or more additional amino acid residues.

With regard to formulae Va to Vc above, it is preferred that R₂₂ is Gly, R₂₃ is absent, R₂₆ is Gly or Ser, R₂₇ is Ser, R₂₈ is Asn and R₂₉ is Met.

Preferred bovine sequences of prion proteins according to formulae Va to Vc are given below: 
Seq. I.D. No: 23
X-(Pro-Gly-Gly-Gly)-Trp-Asn-Thr-Gly-Gly-Ser-Arg-Tyr-
Pro-Gly-Gln-Gly-Ser-Pro-Gly-Gly-Asn-Arg-Tyr-Pro-Pro-
Gln-Gly-(Gly-Gly-Gly-Trp)-Y;
 
Seq. I.D. No: 24
X-(Gly-Gly-Gly-Trp)-Gly-Gln-Pro-His-Gly-Gly-Gly-Trp-
(Gly-Gln-Pro-His)-Y;
 and

Seq. I.D. No: 25
X-(Gly-Gly-Gly-Trp)-Gly-Gln-Gly-Gly-Thr-His-Gly-Gln-
Trp-Asn-Lys-Pro-Ser-Lys-Pro-Lys-Thr-Asn-Met-Lys
(-His-Val-Ala-Gly)-Y.

Preferred sequences of formulae Va to Vc relating to ovine prion proteins are as follows: 
Seq. I.D. No: 26
X-(Pro-Gly-Gly-Gly)-Trp-Asn-Thr-Gly-Gly-Ser-Arg-Tyr-
 
Pro-Gly-Gln-Gly-Ser-Pro-Gly-Gly-Asn-Arg-Tyr-Pro-
Pro-Gln-Gly-(Gly-Gly-Gly-Trp)-Y;
 
Seq. I.D. No: 27
X-(Gly-Gly-Gly-Trp)-Gly-Gln-Pro-His-Gly-Gly-
Gly-Trp-(Gly-Gln-Pro-His)-Y;
 and

Seq. I.D. No: 28
X-(Gly-Gly-Gly-Trp)-Gly-Gln-Gly-Gly-Ser-His-
Ser-Gln-Trp-Asn-Lys-Pro-Ser-Lys-Pro-Lys-Thr-
Asn-Met-Lys(-His-Val-Ala-Gly)-Y.

Preferred sequences of formulae Va to Vc relating to human prion proteins are as follows: 
Seq. I.D. No: 29
X-(Pro-Gly-Gly-Gly)-Trp-Asn-Thr-Gly-Gly-Ser-Arg-Tyr-Pro-
Gly-Gln-Gly-Ser-Pro-Gly-Gly-Asn-Arg-Tyr-Pro-Pro-
Gln-Gly-(Gly-Gly-Gly-Trp)-Y;
 
Seq. I.D. No: 30
X-(Gly-Gly-Gly-Trp)-Gly-Gln-Pro-His-Gly-Gly-Gly-
Trp-(Gly-Gln-Pro-His)-Y;
 and

Seq. I.D. No: 31
X-(Gly-Gly-Gly-Trp)-Gly-Gln-Gly-Gly-Gly-Thr-His-Ser-
Gln-Trp-Asn-Lys-Pro-Ser-Lys-Pro-Lys-Thr-Asn-Met-Lys
(-His-Met-Ala-Gly)-Y.

Particularly preferred sequences of formulae Va to Vc consist of: 
Seq. I.D. No: 49

Gly-Gly-Trp-Asn-Thr-Gly-Gly-Ser-Arg-Tyr-Pro-Gly-Gln-
Gly-Ser-Pro-Gly-Gly-Asn-Arg-Tyr-Pro-
Pro-Gln-Gly-Gly-Gly-Cys;
 Seq. I.D. No: 46 
Gly-Gln-Pro-His-Gly-Gly-Gly-Trp-Gly-Gln-Pro-His-Gly-Gly-
Gly-Trp-Gly-Gln-Pro-His-Gly-Gly-Gly-Trp-Gly-Cys;
 and

Seq. I.D. No: 47

Gly-Gln-Gly-Gly-Ser-His-Ser-Gln-Trp-Asn-Lys-Pro-
Ser-Lys-Pro-Lys-Thr-Asn-Met-Lys-His-Val-Gly-Cys.

We have noted that in the nucleic acid sequence corresponding to region E, it is possible for the repeating sequence of formula Vb to have undergone a frame shift of either +1 or -1. Such frame shifts give rise to altered sequences in region E of the prion protein and our invention provides a synthetic polypeptide having a sequence wherein a repeat in region E has undergone a -1 frame shift as given in formula VI:

X-(R31-R32-Trp-R33)-Trp-Leu-Gly-R34-R35-R36-Trp-R37
(Trp-Leu-Gly-R38)-Y
(VI)

Wherein R₃₁ and R₃₅ are each independently either Ala or Thr; R₃₂ and R₃₆ are each independently an amino acid residue selected from Ser, Pro and Thr; R₃₃ and R₃₇ are each independently either Trp or Arg; R₃₄ and R₃₈ are each independently an amino acid residue selected from Ala, Ser, Pro and Thr; one or more residues within brackets maybe present or absent with the proviso that if they are present they are attached to the rest of the peptide in sequence; and X and Y may each independently be absent or independently be one or more, additional amino acid residues.

With regard to -1 frame shifts in respect of region E in bovines, it is preferred that R₃₁ is Ala, R₃₂, R₃₄, R₃₆ and R₃₈ are each independently either Ser or Pro, R₃₃ and R₃₇ are Arg and R₃₅ is Ala.

It should be noted that preferred sequences for -1 frame shifts in region E of ovines differ in some respects to those given for bovines and in a preferred ovine sequence R₃₁, R₃₂, R₃₃, R₃₅, R₃₆ and R₃₇ correspond to the definitiions given for formula VI above; and R₃₄ and R₃₈ are each independently selected from Ser, Pro and Thr.

In a preferred human sequence according to formula VI R₃₁, R₃₄, R₃₅ and R₃₈ are each Ala, R₃₂ and R₃₆ are each independently either Ser or Pro and R₃₃ and R₃₇ are both Trp.

As mentioned previously, the frame shift may be +1 in the repeat portion of region E and this gives rise to different amino acid sequences. Accordingly, our invention provides a synthetic polypeptide according to formula VII below which relates to a +1 frame shift in the repeat of region E:

X-(R39-R40-Met-R41)-Val-Ala-Gly-R42-R43-R44-Met-R45-
(Val-Ala-Gly-R46)-Y
(VII)

Wherein R₃₉ and R₄₃ are each independently either Ser or Asn; R₄₀ and R₄₄ are each independently an amino acid residue selected from Pro, Leu and His, R₄₁ and R₄₅ are each independently Val or Glu; R₄₂ and R₄₆ are each independently selected from Val, Ala, Asp and Gly; one or more residues within brackets maybe present or absent with the proviso that if they are present they are attached to the rest of the peptide in sequence; and X and Y may each independently be absent or independently be one or more, additional amino acid residues.

A preferred bovine sequence according to formula VII comprises R₃₉ and R₄₃ each being Ser, R₄₂ and R₄₆ each being independently either Val or Ala and R₄₄ being either Pro or Leu; with the other R groups being as defined in formula VII.

A preferred sequence according to formula VII relating to ovines is the same as given in general formula VII except R₄₂ and R₄₆ are each independently selected from Val, Ala and Asp.

With regard to a preferred human sequence according to formula VII, R₃₉ and R₄₃ are Ser, R₄₀ and R₄₄ are each independently Pro or Leu, R₄₁ and R₄₅ are Val and R₄₂ and R₄₆ are each independently either Asp or Gly.

Our invention also provides a synthetic peptide sequence relating to region F and having either the general formula VIIIa or VIIIb: 
X-(Asn-Phe-Val-His)-Asp-Cys-Val-Asn-Ile-Thr-R47-Lys-
R48-His-Thr-Val-R49-Thr-Thr-Thr-Lys-Gly-Glu-Asn-
Phe-Thr-Glu-(Thr-Asp-R50-Lys)-Y
(VIIIa)
 
X-(Met-Cys-R51-Thr)-Gln-Tyr-R52-R53-Glu-Ser-Gln-Ala-
Tyr-Tyr-R54-R55-Arg-(R56-R57-Ser-R58-R59)-Y
(VIIIb)
 Wherein
R₄₇ is either Ile or Val;
R₄₈ and R₅₂ are each independently either Gln or Glu;
R₄₉ is either Val or Thr;
R₅₀ is either Val or Ile;
R₅₁ is an amino acid residue selected from Ile, Thr and Val;
R₅₂ is Gln or Glu:
R₅₃ is either Arg or Lys;
R₅₄ is either Asp or Gln;
R₅₅ is Gly or is absent;
R₅₆ is either Gly or Arg;
R₅₇ is either Ala or Ser;
R₅₈ is Ser or absent;
R₅₉ is an amino acid residue selected from Ala, Thr, Met and Val;
one or more residues within brackets maybe present or absent with the proviso that if they are present they are attached to the rest of the peptide in sequence; and X and Y may each independently be absent or independently be one or more, e.g. 3, additional amino acid residues.

It is preferred in formula VIIIa that R₄₉ is Thr and in formula VIIIb that R₅₁ is Ile, R₅₃ is Arg, R₅₄ is Gln, R₅₅ is absent, R₅₆ is Gly, R₅₇ is Ala and R₅₈ is absent.

Most preferred bovine, ovine and human sequences according to formulae VIIIa and VIIIb are given below in order: 
Seq. I.D. No: 32
X-(Asn-Phe-Val-His)-Asp-Cys-Val-Asn-Ile-Thr-Val-Lys-
Glu-His-Thr-Val-Thr-Thr-Thr-Thr-Lys-Gly-Glu-Asn-
Phe-Thr-Glu-(Thr-Asp-Ile-Lys)-Y
bovine (VIIIa),
 and 
Seq. I.D. No: 33
X-(Met-Cys-Ile-Thr)-Gln-Tyr-Gln-Arg-Glu-Ser-Gln-Ala-
Tyr-Tyr-Gln-Arg-(Gly-Ala-Ser-Val)-Y
bovine (VIIIb);
 
Seq. I.D. No: 34
X-(Asn-Phe-Val-His)-Asp-Cys-Val-Asn-Ile-Thr-Val-Lys-
Gln-His-Thr-Val-Thr-Thr-Thr-Thr-Lys-Gly-Glu-Asn-
Phe-Thr-Glu-(Thr-Asp-Ile-Lys)-Y
ovine (VIIIa),
 and 
Seq. I.D. No:35
X-(Met-Cys-Ile-Thr)-Gln-Tyr-Gln-Arg-Glu-Ser-Gln-Ala-
Tyr-Tyr-Gln-Arg-(Gly-Ala-Ser-Val)-Y
ovine (VIIIb);
 
Seq. I.D. No:36
X-(Asn-Phe-Val-His)-Asp-Cys-Val-Asn-Ile-Thr-Ile-Lys-
Gln-His-Thr-Val-Thr-Thr-Thr-Thr-Lys-Gly-Glu-Asn-
Phe-Thr-Glu-(Thr-Asp-Val-Lys)-Y
human (VIIIa),
 and

Seq. I.D. No:37

X-(Met-Cys-Ile-Thr)-Gln-Tyr-Glu-Arg-Glu-Ser-Gln-Ala-
Tyr-Tyr-Gln-Arg-(Gly-Ser-Ser-Met)-Y
human (VIIIb).

Particularly preferred sequences according to formula VIIIa and VIIIb are selected from 
Seq. I.D. No: 50

Val-Asn-Ile-Thr-Val-Lys-Gln-His-Thr-Val-Thr-Thr-Thr-Thr-
Lys-Gly-Glu-Asn-Phe-Thr-Glu-Gly-Cys;
 and

Seq. I.D. No: 48

Cys-Ile-Thr-Gln-Tyr-Gln-Arg-Glu-
Ser-Gln-Ala-Tyr-Tyr-Gln-Arg.

Synthetic polypeptides according to any one of formulae I to VIIIb above without X and Y being present will of course be useful, for example, in the production of antibodies. However, when X or Y are present they may be any length but preferably less than 20 amino acids, more preferably less than 10, eg. 3 to 6. It will of course be appreciated that a sequence according to any one of formulae I to VIIIb may constitute a protein with X and Y being major portions of the protein with the antigenic sequence being for example, part of an exposed loop on a globular protein.

It is preferred that if X or Y are present they are relatively short sequences, typically 1 to 3 residues long. In most instances X is preferably absent and Y is 1 or 2 residues long, e.g. -Cys or -Gly-Cys.

All the sequences herein are stated using the standard I.U.P.A.C. three-letter-code abbreviations for amino acid residues defined as follows: Gly-Glycine, Ala-Alanine, Val-Valine, Leu-Leucine, Ile-Isoleucine, Ser-Serine, Thr-Threonine, Asp-Aspartic acid, Glu-Glutamic acid, Asn-Asparagine, Gln-Glutamine, Lys-Lysine, His-Histidine, Arg-Arginine, Phe-Phenylalanine, Tyr-Tyrosine, Trp-Tryptophan, Cys-Cysteine, Met-Methionine and Pro-Proline.

Polypeptides according to the invention may be used to raise antibodies which will cross-react with prion proteins produced in a wide range of organisms. Our analyses have shown that since the conformational, topographic and electrostatic properties of polypeptides according to the invention are such that they are highly likely to elicit the production of antibodies which will cross-react with prion proteins from several or many organisms, further advantages may arise from combining several variant polypeptides in a larger polypeptide. Such a polypeptide may have the general formula (IX):

[Lₐ-F]ₘ - [L_{b}-G]ₙ - L_{c} (IX)

wherein F and G may each independently be a polypeptide or sub-fragment according to any one of Formulae I to VIIIb, L is a linking sequence, a, b and c are each independently 0 or 1 and m and n are each positive numbers e.g. between 1 and 10 inclusive. L is preferably a short, conformationally flexible section of polypeptide chain such as, for example and without limit (Seq. I.D. No: 38) Gly-Gly-Gly-Gly-Gly, (Seq. I.D. No: 39) Gly-Pro-Gly-Pro-Gly-Pro or (Seq. I.D. No: 40) Gly-Ser-Ala-Gly-Ser-Gly-Ala. It should be clear that each repeat may optionally have a different variant of a polypeptide according to the invention.

It should be noted certain of the C-teminals correspond to N-terminals, particularly formula Va to formula Vb, formula Vc to formula I, formula I to formula II, formula II to formula III, formula III to formula VIIIa and formula VIIIb to formula IV. Advantage may be taken to this correspondence when producing larger polypeptides according to formula IX. Linking sequences together with respective X and Y moieties may be omitted and residues in brackets may, be selected so that either the regions of correspondence are duplicated or some or all of the duplicated residues are omitted. In the latter case it will be seen that the C-terminal of one polypeptide merges with the N-terminal of the other polypeptide.

Polyvalent determinant analogues as defined by Formula IX may be either what is referred to as pseudohomopolyvalent, wherein variants of essentially the same determinant analogue are repeated in a single polypeptide chain and/or heteropolyvalent, wherein distinct determinants are included in a single polypeptide. In addition, simple homopolyvalent polypeptide immunogens, which contai.. multiple copies of the same variant of one of the determinant analogues according to any one of formulae I to VIIIb, would also be expected to be effective, and are also included within the scope of the present invention.

It is to be understood that any antigenically significant subfragments and/or antigenically significant variants of the above-identified polypeptide sequences which retain the general form and function of the parent polypeptide are included within the scope of this invention. In particular, the substitution of any of the specific residues by residues having comparable conformational and/or physical properties, including substitution by rare (but naturally occurring, e.g. D-stereoisomers) or synthetic amino acid analogues, is included. For example, substitution of a residue by another in the same Set, as defined below, is included within the ambit of the invention; Set 1 - Ala, Val, Leu, Ile, Phe, Tyr, Trp and Met; Set 2 - Ser, Thr, Asn and Gln; Set 3 - Asp and Glu; Set 4 - Lys, His and Arg; Set 5 - Asn and Asp; Set 6 - Glu and Gln; Set 7 - Gly, Ala, Pro, Ser and Thr. D-stereoisomers of all amino acid types, may be substituted, for example, D-Phe, D-Tyr and D-Trp.

Di Martino et al. (J. Molecular Recognition 4 85-91 (1991)) describes the production of polyclonal antibodies to two synthetic polypeptides of mouse PrP. Bolton et al. (J. Virology 65, 3667-3675 (1991)) describes epitope mapping by means of synthetic polypeptides of monoclonal antibodies raised to hamster scrapie agent.

In preferred embodiments of the invention, X and Y if present may independently include one or more segments of protein sequence with the ability to act as a T-cell epitope. For example, segments of amino acid sequence of the general formula 1-2-3-4, where 1 is Gly or a charged amino acid (e.g. Lys, His, Arg, Asp or Glu), 2 is a hydrophobic amino acid (e.g. Ile, Leu, Val, Met, Tyr, Phe, Trp, Ala), 3 is either a hydrophobic amino acid (as defined above) or an uncharged polar amino acid (e.g. Asn, Ser, Thr, Pro, Gln, Gly), and 4 is a polar amino acid (e.g. Lys, Arg, His, Glu, Asp, Asn, Gln, Ser, Thr, Pro), appear to act as T-cell epitopes in at least some instances (Rothbard, J.B. & Taylor, W.R. (1988). A sequence pattern in common to T-cell epitopes. The EMBO Journal 7(1): 93-100). Similarly segments can be of the sequence 1'-2'-3'-4'-5', wherein 1' is equivalent to 1 as defined earlier, 2' to 2, 3' and 4' to 3, and 5' to 4 (ibid). Both forms are included within the scope of the present invention and one or more T-cell epitopes (preferably less than five) which may be of the type defined above or may be of other structure and which may be separated by spacer segments of any length or composition, preferably less than five amino acid residues in length and comprising for example residues selected from Gly, Ala, Pro, Asn, Thr, Ser or polyfunctional linkers such as non-a amino acids. It is possible for a C- or N-terminal linker to represent a complete protein, thus obviating the possible need for conjugation to a carrier protein.

Also included within the scope of this invention are derivatives of the polypeptides according to any one formulae I to VIIIb in which X or Y are or include a "retro-inverso" amino acid, i.e. a bifunctional amine having a functional group corresponding to an amino acid. For example an analogue according to the invention and containing a retro-inverso amino acid may have the formula: 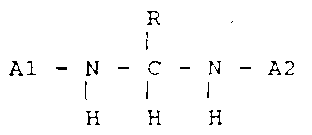 where R is any functional group, e.g. a glycine side chain, and A1 and A2 are preferably each a copy of one of the analogues defined herein (but not necessarily the same) attached by its N- or C-terminal end. T-cell epitopes may optionally be included as discussed earlier.

Retro-inverso modification of peptides involves the reversal of one or more peptide bonds to create analogues more resistant than the original molecule to enzymatic degradation and offer one convenient route to the generation of branched immunogens which contain a high concentration of epitope for a medium to large immunogen. The use of these compounds in large-scale solution synthesis of retro-inverso analogues of short-chain biologically active peptides has great potential.

Peptides according to the invention may be synthesised by standard peptide synthesis techniques, for example using either standard 9-fluorenylmethoxycarbonyl (F-Moc) chemistry (see, for example, Atherton, E. and Sheppard, R. C. (1985) J. Chem. Soc. Chem. Comm. 165) or standard butyloxycarbonate (T-Boc) chemistry although it is noted that, more recently, the fluorenylmethoxycarbonyl (Fmoc)/tert-butyl system, developed by Sheppard et al has found increasingly wide application (Sheppard, R.C.1986 Science Tools, The LKB Journal 33, 9). The correctness of the structure and the level of purity, which will normally be in excess of 85%, should be carefully checked, and particular attention be given to the correctness of internal disulphide bridging arrangements when present. Various chromatographic analyses, including high performance liquid chromatography, and spectrographic analyses, including Raman spectroscopy, may for example be employed for this purpose.

It is to be understood that the polypeptides according to the invention may be synthesised by any conventional method, either directly using manual or automated peptide synthesis techniques as mentioned above, or indirectly by RNA or DNA synthesis and conventional techniques of molecular biology and genetic engineering. Such techniques may be used to produce hybrid proteins containing one or more of the polypeptides inserted into another polypeptide sequence.

Another aspect of the present invention therefore provides a DNA molecule coding for at least one synthetic polypeptide according to the invention, preferably incorporated into a suitable expression vector replicable in microorganisms or in mammalian cells. The DNA may also be part of the DNA sequence for a longer product e.g. the polypeptides may be expressed as parts of other proteins into which they have been inserted by genetic engineering. One practical guide to such techniques is "Molecular cloning: a laboratory manual" by Sambrook, J., Fritsch, E.F. and Maniatis, T. (2nd Edition, 1989).

It should be noted that analogues incorporating retro-inverso amino acid derivatives cannot be made directly using a recombinant DNA system. However, the basic analogues can, and they can then be purified and chemically linked to the retro-inverso amino-acids using standard peptide/organic chemistry. A practical and convenient novel procedure for the solid-phase synthesis on polyamide-type resin of retro-inverso peptides has been described recently [Gazerro, H., Pinori, M. & Verdini, A.S. (1990). A new general procedure for the solid-phase synthesis of retro-inverso peptides. In "Innovation and Perspectives in Solid Phase Synthesis" Ed. Roger Epton. SPCC (UK) Ltd, Birmingham, UK].

The polypeptides are optionally linked to a carrier molecule, either through chemical groups within the polypeptides themselves or through additional amino acids added at either the C- or N-terminus, and which may be separated from the polypeptides themselves or surrounded by one or more additional amino acids, in order to render them optimal for their immunological function. Many linkages are suitable and include for example use of the side chains of Tyr, Cys and Lys residues. Suitable carriers include, for example, purified protein derivative of tuberculin (PPD), tetanus toxoid (TT), cholera toxin and its B subunit, ovalbumin, bovine serum albumin (BSA), soybean trypsin inhibitor (STI), muramyl dipeptide (MDP) and analogues thereof, diphtheria toxoid (DPT), keyhole limpet haemocyanin (KLH) and Braun's lipoprotein although other suitable carriers will be readily apparent to the skilled person. For example, multiple antigen peptides may be used such as those comprising a polylysyl core, e.g. heptalysyl, bearing reactive amine termini. Polypeptide antigens according to the invention may be reacted with, or synthesised on, the amino termini and different polypeptide antigens may be reacted with the same core or carrier. When using PPD as a carrier for polypeptides according to the invention, a higher titre of antibodies is achieved if the recipient of the polypeptide-PPD conjugate is already tuberculin sensitive, e.g. by virtue of earlier BCG vaccination. In the case of a human vaccine it is worth noting that in the UK and many other countries the population is routinely offered BCG vaccination and is therefore largely PPD-sensitive. Hence PPD is expected to be a preferred carrier for use in such countries.

The mode of coupling the polypeptide to the carrier will depend on the nature of the materials to be coupled. For example, a lysine residue in the carrier may be coupled to a C-terminal or other cysteine residue in a polypeptide by treatment with N-γ -maleimidobutyryloxy-succinimide (Kitagawa, T. & Ackawa, T. (1976) J. Biochem. 79, 233). Alternatively, a lysine residue in the carrier may be conjugated to a glutamic or aspactic acid residue in the peptide using isobutylchloroformate (Thorell, J.I. De Larson, S.M. (1978) Radioimmunoassay and related techniques: Methodology and clinical applications, p.288). Other coupling reactions and reagents have been described in the literature.

The polypeptides, either alone or linked to a carrier molecule, may be administered by any route (eg parenteral, nasal, oral, rectal, intra-vaginal), with or without the use of conventional adjuvants (such as aluminium hydroxide or Freund's complete or incomplete adjuvants) and/or other immunopotentiating agents. The invention also includes formulation of polypeptides according to the invention in slow-release forms, such as a sub-dermal implant or depot comprising, for example, liposomes (Allison, A.C. & Gregoriadis, G. (1974) Nature (London) 252, 252) or biodegradable microcapsules manufactured from co-polymers of lactic acid and glycolic acids (Gresser, J. D. and Sanderson, J. E. (1984) in "Biopolymer Controlled Release Systems" pp 127-138, Ed. D. L. Wise).

Polypeptides according to the invention may be used either alone or linked to an appropriate carrier, as:
(a) As ligands in assays of, for example, serum from patients or animals;
(b) Peptide vaccines, for use in prophylaxis;
(c) As quality control agents in testing, for example, binding levels of antibodies raised against the polypeptides;
(d) As antigenic agents for the generation of monoclonal or polyclonal antibodies by immunisation of an appropriate animal, such antibodies being of use for (i) the scientific study of prion proteins, (ii) as diagnostic agents, e.g. as part of immunohistochemical reagents, (iii) for the passive immunisation of animals or patients, either as a treatment for encephalophathies or in combination with other agents, (iv) as a means of targeting other agents to regions comprising prion proteins, such agents either being linked covalently or otherwise associated, e.g. as in liposomes containing such agents and incorporating antibodies raised against any of the antigenic polypeptides and (v) for use as immunogens to raise anti-idiotype antibodies; such anti-idiotype antibodies also form part of this invention. The invention further provides for genetically engineered forms or sub-components, especially V_{H} regions, of antibodies raised against the polypeptides, and of ovinised, bovinised, or humanised forms of antibodies initially raised against the polypeptides in other animals, using techniques described in the literature; and
(e) The treatment of encephalopathies, either by displacing the binding of prion proteins to human or animal cells or by disturbing the three-dimensional organisation of the protein in vivo; as well as aiding the scientific study of prion proteins in vitro.

In respect of detection and diagnosis, of prion proteins or antibodies against prion proteins, the skilled person will be aware of a variety of immunoassay techniques known in the art, inter alia, sandwich assay, competitive and non-competitive assays and the use of direct and indirect labelling.

A further aspect of the invention provides a kit for detecting prion proteins or antibodies against prion proteins which comprises at least one synthetic polypeptide according to the invention.

The preparation of polyclonal or monoclonal antibodies, humanised forms of such antibodies (see, for example, Thompson K. M. et al (1986) Immunology 58, 157-160), single domain antibodies (see, for example, Ward, E. S., Gussow, D., Griffiths, A. D., Jones, P. and Winter, G. (1989) Nature 341, 544-546), and antibodies which might cross the.blood-brain barrier, which bind specifically to a synthetic polypeptide according to the present invention, may be carried out by conventional means and such antibodies are considered to form part of this invention. Antibodies according to the invention are, inter alia, of use in a method of diagnosing mammalian encephalopathies which comprises incubating a sample of tissue or body fluid of mammal with an amount of antibody as described herein and determining whether, and if desired the extent to which and/or rate at which, cross-reaction between said sample and said antibody occurs. Diagnostic kits which contain at least one of said antibodies also form part of this invention.

A further aspect of the invention provides synthetic polypeptides for use in therapy or prophylaxis of mammalian encephalopathies and/or stimulating the mammalian immune system and/or blocking the cellular binding or aggregation of the prion proteins and for the preparation of medicaments suitable for such uses. Also included are pharmaceutical compositions containing, as active ingredient, at least one polypeptide or polypeptide-carrier conjugate as described herein in association with one or more pharmaceutically acceptable adjuvants, carriers and/or excipients. The compositions may be formulated for oral, rectal, nasal or especially parenteral administration (including intra-CNS administration).

The invention further provides a method of therapy or prophylaxis of mammalian encephalopathies and/or of stimulating the mammalian immune system and/or of blocking the cellular binding or aggregation of the prion proteins, which comprises administering an amount of a polypeptide as hereinbefore defined, either in isolation or in combination with other agents for the treatment of encephalopathies.

Discrimination between natural PrP^{C} and PrP^{SC} is highly desired since PrP^{C} is found in normal subjects and both PrP^{C} and PrP^{SC} are found in a diseased subject. We have found that peptide sequences according to the invention, preferably those relating to regions A, B and C, and significant sub-fragments thereof may be used to discriminate between natural PrP^{C} and infective PrP^{SC}. Also, antibodies raised against these peptide sequences and sub-fragments and the nucleotide sequences which code for such peptide sequences and sub-fragments may also be used to discriminate between PrP^{C} and PrP^{SC}. Accordingly, the invention provides a method of discriminating between PrP^{C} and PrP^{SC} in which a sample is contacted with a substance selected from peptide sequences according to the invention, preferably those relating to regions A, B and C, and significant sub-fragments thereof, antibodies raised against said sequences and sub-fragments and the presence or absence of PrP^{SC} is determined.

In some instances discrimination may be enhanced by pretreatment of the sample, for example by pre-digestion with enzymes e.g. proteinase K, or denaturation by strong alkali e.g. 6M guanidine hydrochloride or by a combination of such treatments.

It will be preferable to use the peptide sequences, antibodies and nucleotide sequences which relate to the specific subject under test, e.g. bovine sequences and antibodies for cattle, ovine sequences and antibodies for sheep.

It may be advantageous to immunise with a cocktail containing (i) a given analogue conjugated to more than one type of carrier molecule, and/or (ii) more than one kind of analogue conjugated to the same carrier molecule. Moreover, any of the peptide analogues, their conjugates, and cocktails thereof may be administered in any suitable adjuvant or delivery system, and more than one adjuvant or delivery system may be combined to form a so-called "super-cocktail". Preferred adjuvants and delivery systems include aluminium hydroxide (alum), liposomes, micelles, niosomes, ISCOMS, Brauns lipoprotein and whole-cell or components of microbial animal vaccines.

### Example 1

A preferred bovine form of formula II (Seq. I.D. No: 41) Ala-Met-Ser-Arg-Pro-Leu-Ile-His-Phe-Gly-Ser-Asp-Tyr-Glu-Asp-Arg-Tyr-Tyr-Arg-Glu-Asn-Met-His-Arg-Gly-Cys (related to Seq. I.D. No: 7) in which the C-terminal Y extension is Gly-Cys according to the invention is synthesised using standard solid-phase Fmoc methodologies. The peptide is cleaved from the resin in the presence of trifluroacetic acid and subsequent purification is achieved by gel filtration, ion exhange chromatography and reverse phase high performance liquid chromatography. The peptide is conjugated to a variety of carriers by MBS (m-Maleimido-benzoyl-N-hydroxy succinimide ester), a well-known hetero-bifunctional reagent.

Examples of carriers include KLH, BSA and TT which have been shown to provide necessary immunopotentiating properties to B cell epitopes.

The peptide carrier conjugates are emulsified in Freund's Complete Adjuvant and are administered intramuscularly to mice. Subsequent booster injections are given in Freund's Incomplete Adjuvant.

The ensuing serum antibody response is monitored throughout the immunisation schedule by enzyme immunoassay (ELISA) using immobilised antigen (formula II), coupled to BSA, the serum sample under test, and an enzyme-labelled anti-mouse antibody.

In this example, use of carriers, adjuvants and delivery systems and booster injections are effected in order to determine an optimal protocol for producing anti-formula II antibodies.

### Example 2

Antibodies to formula II are used as diagnostic reagents for assaying the presence of prion protein in serum, in "cell carriers" in serum and in tissue biopsies of injected animal species.

A direct enzyme immunoassay (ELISA) can detect the presence of extracted prion protein by its immobilisation onto a solid substrate. Reaction of mouse antisera raised to formula II with native prion protein is detected with an enzyme-labelled anti-mouse antiserum. The reaction is quantified by addition of a suitable substrate and reading the optical density of the colour produced.

Furthermore, immunohistochemical diagnosis of prion proteins in tissue biopsies can be performed by reacting anti-formula II antibodies with paraffin wax embedded or frozen tissue. Reactions can be detected using a standard indirect immunoperoxidase technique.

### Example 3

### MATERIALS AND METHODS

### Peptide Synthesis

The following peptides were synthesised using standard solid-phase Fmoc methodologies.

Peptide II: (Seq. I.D. No: 42)

Ser-Ala-Met-Ser-Arg-Pro-Leu-Ile-His-Phe-Gly-
Asn-Asp-Tyr-Glu-Asp-Arg-Tyr-Tyr-Gly-Cys

(A preferred ovine sub-fragment of formula II).

Peptide BII: (Seq. I.D. No: 43)

Ser-Ala-Met-Ser-Arg-Pro-Leu-Ile-His-Phe-Gly-
Ser-Asp-Tyr-Glu-Asp-Arg-Tyr-Tyr-Gly-Cys

(A preferred bovine sub-fragment of formula II).

Peptide III: (Seq. I.D. No: 44)

Asn-Met-Tyr-Arg-Tyr-Pro-Asn-Gln-Val-Tyr-Tyr-Arg-Pro-Val-
Asp-Arg-Tyr-Ser-Asn-Gln-Asn-Asn-Phe-Val-His-Gly-Cys

(A preferred ovine sequence of formula III (p8, ln 30-32).

Peptide BIII: (Seq. I.D. No: 45)

Asn-Met-His-Arg-Tyr-Pro-Asn-Gln-Val-Tyr-Tyr-Arg-Pro-Val-
Asp-Gln-Tyr-Ser-Asn-Gln-Asn-Asn-Phe-Val-His-Gly-Cys

(A preferred bovine sequence of formula III (p8, ln 26-28).

Peptide Vb: (Seq. I.D. No: 46)

Gly-Gln-Pro-His-Gly-Gly-Gly-Trp-Gly-Gln-Pro-His-Gly-Gly-
Gly-Trp-Gly-Gln-Pro-His-Gly-Gly-Gly-Trp-Gly-Cys

(A preferred ovine/bovine sequence of formula Vb).

Peptide Vc: (Seq. I.D. No: 47)

Gly-Gln-Gly-Gly-Ser-His-Ser-Gln-Trp-Asn-Lys-Pro-
Ser-Lys-Pro-Lys-Thr-Asn-Met-Lys-His-Val-Gly-Cys

(A preferred ovine sequence of formula Vc).

Peptide VIIIb: (Seq. I.D. No: 48)
Cys-Ile-Thr-Gln-Tyr-Gln-Arg-Glu-
Ser-Gln-Ala-Tyr-Tyr-Gln-Arg

(A preferred ovine/bovine sequence of formula VIIIb).

Peptide Va: (Seq. I.D. No: 49)
Gly-Gly-Trp-Asn-Thr-Gly-Gly-Ser-Arg-Tyr-
Pro-Gly-Gln-Gly-Ser-Pro-Gly-Gly-Asn-Arg-Tyr-Pro-
Pro-Gln-Gly-Gly-Gly-Cys

Pep[?]ide VIIIa: (Seq. I.D. No: 50)

Val-Asn-Ile-Thr-Val-Lys-Gln-His-Thr-Val-Thr-Thr-Thr-Thr-
Lys-Gly-Glu-Asn-Phe-Thr-Glu-Gly-Cys

(A preferred ovine sequence of formula VIIIa).

Peptide I: (Seq. I.D. No: 51)
Lys-His-Met-Ala-Gly-Ala-Ala-Ala-Ala-Gly-Ala-
Val-Val-Gly-Gly-Leu-Gly-Gly-Tyr-Met-Leu-Gly-Ser-Ala-
Met-Ser-Arg-Gly-Cys.

Peptides I, II, BII, III, BIII a, Vb, Vc and VIIIa were synthesised with the C-terminal extension according to the invention. The peptides were cleaved from the resin in the presence of trifluoroacetic acid and subsequent purification was achieved by reverse phase high performance liquid chromatography. All peptides had a purity of 85% or more.

### Conjugation of peptides to ovalbumin

Peptides were conjugated through their C-terminal (peptides II, BII, III, BIII Vb and Vc) or N-terminal (peptide VIIIb) Cys residues. Peptides were dissolved in dimethyl sulphoxide (DMSO) to a concentration of 10 mg/ml. Preactivated ovalbumin (Pierce, Imject Kit) was resuspended in 1 ml of distilled water, and equal volumes of preactivated ovalbumin and peptide were mixed and the solution allowed to stand at room temperature for 3 hours. The conjugate was dialysed overnight against phosphate buffered saline (PBS) to remove the DMSO and unconjugated peptide.

The extent of conjugation was determined by measuring the free-thiol content using an Ellman's assay and by monitoring the increase in the molecular mass of the conjugate by SDS-PAGE (sodium dodecyl sulphate-polyacrylamide gel electrophoresis).

### Generation of rabbit antisera.

Antiserum was raised against each of the peptide conjugates in two female New Zealand White rabbits. Each rabbit received an amount of conjugate equivalent to 40 µg of peptide for both the primary inoculation and the boosters. Rabbits were injected as follows:
- Day 0:: Conjugate in Freund's Complete Adjuvant (1:1, v/v) intramuscularly.
- Day 21:: Conjugate in Freund's Incomplete Adjuvant (1:1, v/v) intramuscularly.
- Day 31:: Conjugate on its own intraperitoneally.

Animals were bled on day 41, and the sera assayed for anti-peptide antibody by ELISA (using free peptide as the coating antigen). The sera were also used in immunoblot and dot blot assays to see if they could recognise proteins from the brain homogenates.

### Preparation of brain homogenates

Scrapie-free brain material was obtained from a flock of New Zealand sheep in quarantine.

Scrapie-infected brain material was obtained from a Department of Agriculture and had been histopathalogically diagnosed as being scrapie infected.

BSE-infected brain material was obtained via a government Agriculture Department and had been histopathalogically certified as being BSE infected.

BSE-free material was obtained through a private source.

Ha27-30 is brain material obtained from an inbred hamster scrapie model, which has been shown to contain a high level of the scrapie-infective agent. It was used as a positive control.

Small samples of infected and uninfected brain were weighed and 10% (w/v) homogenates made up in 10% (v/v) solution of Sarkosyl in 25 mM Tris-HCl pH 7.4 (homogenisation buffer). The homogenate was incubated at 4°C for 30 mins and then spun at 6000 x g for 30 mins. The supernatant was collected and the protein content determined using the BCA protein assay kit (Pierce). The protein concentration was adjusted to 3 mg/ml using homogenisation buffer.

### ELISA (Enzyme-linked immunosorbent assay)

A 8 µM solution of free peptide in PBS was used as the coating antigen. Microtitre plates were coated by adding 50 µl of the antigen concentration to each well and then incubating for 1 hour at 37°C to allow binding to occur. Each well was washed 5 times, for 2 minutes, with 300 µl of PBS containing 0.05% Tween 20. After washing, the plates were blocked by incubating for 1 hour at 37°C with PBS containing 0.3% Tween 20 and 3% non-fat milk. An aliquot of 50 µl of primary antibody (i.e. antisera) diluted in PBS was added to the appropriate wells and the plates incubated for 1 hour at 37°C. Plates were washed as before, and then incubated with Horseradish peroxidase conjugated swine anti-rabbit immunoglobulin (anti Ig/HRP) at a dilution of 1:1000 in PBS for 1 hour at 37°C. The plates were washed and 50 µl of OPD (O-phenylenediamine dihydrochloride substrate (10 mg/ml) in citrate buffer) added to each well and the reaction allowed to proceed at room temperature for 10 minutes, before being stopped by the addition of sulphuric acid. The absorbence of each well was measured at 492 nm using an ELISA plate reader. The titres were recorded as the dilutions which gave a positive optical density (OD) reading at least 3 times that of the background. The background was taken as the OD readings from wells which had not been coated with antigen.

### Dot blot detection of PrP in brain homogenates

The brain homogenates prepared as described earlier were diluted 10-fold in PBS, and 100 µl of homogenates (containing 30 µg total protein) were applied to nitrocellulose filters using BRL 96 well vacuum manifold. The filters were dried for 1 hour at room temperature. The filters were then either wet with TBST (10 mM Tris-HCl pH7.4, 150 mM Nacl, 0.05% Tween 20) and PrP detected as described in the immunoblots, or the protein sample further treated. This further treatment of the sample included digestion of the protein on the filter using 100 µg/ml proteinase K in TBST for 90 minutes at room temperature.

The proteinase K was inactivated by the addition of PMSF (phenylmethylsulphonyl fluoride) to a concentration of 5 mM in TBST. After protein digestion, some samples were also denatured by incubating the filters in 6M guanidine HCl containing 5 mM PMSF for 10 minutes. The guanidine was removed by 3 washes with TBST prior to incubation with the primary antibody.

### Immunoblots. (Western Blots)

SDS-PAGE was performed on the brain homogenates, prepared as described previously, using standard techniques. The samples within the gel were transferred onto nitrocellulose in a Biorad transblot using Towbin Buffer (25 mM Tris, 190 mM glycine and 0.1% SDS) at 70 mA overnight. The nitrocellulose filter was blocked with 5% non-fat milk for 30 minutes at room temperature. The primary antibody (i.e. antisera) diluted in TBST was applied for 3 hours at room temperature, the filter washed 3 times for 10 minutes in TBST and the filter incubated for 2 hours at room temperature with the alkaline phosphatase-conjugated swine anti-rabbit immunoglobulin diluted at a dilution of 1:2000. After washing, the protein bands were detected using the NBT/BCIP (nitro-blue tetrazolium; 5-bromo-4-chloro-3-indolyl phosphate) substrate (Boehringer Mannheim).

### RESULTS

1) Antibody titres: Good antibody titres to the peptides were obtained in all cases, though the level varied enormously. The peptide which gave the highest titre, also gave the best results in the dot blots.
2) Dot Blot Data: Uninfected tissue would be expected to contain only normal prion protein (PrP^{c}). Infected tissue would be expected to contain both the normal and the diseased (PrP^{sc}) forms of PrP.
   PrP^{c} has a molecular weight of approximately 33-35 kD.
   PrP^{sc} has a molecular weight of approximately 27-30 kD and is missing an N-terminal segment that is present in the PrP^{c} form. Otherwise, the amino acid sequence of PrP^{sc} is exactly the same as that of PrP^{c}. Probably the most significant characteristic of PrP^{sc} is resistance to enzyme degradation with proteinase K, a non-specific protein-digesting enzyme.
   When a protein sample is treated with proteinase K any PrP^{c} should be completely digested. Therefore, in a sample containing only PrP^{c}, no PrP of any form will remain after proteinase K treatment. However, in a sample containing PrP^{c} and PrP^{sc} (i.e. a diseased sample), PrP^{sc} will remain after treatment.
   There are antibodies currently available which recognise PrP^{sc}, but they only recognise the denatured protein. Therefore after proteinase K treatment, samples in the dot blot test were treated with guanidine HCl, a denaturing agent, so that such antibodies could be used to detect PrP^{sc}.
   The data are given in Tables I-V.

### Peptide II:

Good titres. Dot blots appear to indicate that some discrimination is occurring. Negative results were obtained from the Western blots.

### Peptide III:

Reasonable titres. Possibly there is recognition of a non-specific (perhaps non-protein) component in the proteinase K and guanidine treated samples. Negative results were obtained from the Western blots.

### Peptide Vb:

Good titres. Although it appears that there might be some discrimination occurring, the Vb peptide in fact occurs within the N-terminal region that is missing in PrP^{sc}. One would therefore not expect to see any recognition in the infected material treated with proteinase K and guanidine. However, one possible explanation is that the PrP^{c} present in the infected material has not been completely digested by the proteinase K. Negative results were obtained from the Western blots.

Peptide Vc: Excellent titres. These results are exactly as expected. As mentioned previously, antibodies which recognise PrP^{sc} generally only recognise the protein in its denatured state. Infected and uninfected samples, as well as containing PrP^{sc} and/or PrP^{c} in their "native" states, will also contain both PrP forms in various stages of denaturation due to natural protein turnover within cells. For this reason, antibodies would be expected to detect all three untreated samples. However, proteinase K treatment will digest PrP^{c} and any partially denatured PrP^{sc} leading to a loss of antibody recognition in all samples (assuming the antibody only recognises denatured PrP). The addition of guanidine should restore antibody recognition in material which had originally contained PrP^{sc}. Western blots showed up the expected protein bands at the correct molecular weights.

### Peptide VIIIb:

Reasonable titre. There may be recognition of a non-specific component. Negative results were obtained from the Western blots.

### Peptides BII & BIII:

The titres are reasonable and there are strong positive results from untreated normal and infected bovine brain material.

In summary, good anti-peptide titres obtained in all cases, the Western blots only worked well in the case of peptide Vc, which also gave the highest titre and the dot blots show that there is some discrimination occurring between PrP^{c} and PrP^{sc} with peptide Vc. Data from peptide II also suggests that discrimination is occurring.

**Table I:**

| Results from ovine peptide sequences | | | | | | | | |
|---|---|---|---|---|---|---|---|---|
| | Pept/carrier ratio | Antibody number | Titre | Ovine Brain Material | DOT BLOT | | | West Blot |
| | | | | | Untrt | Prot K | Prot K + Gua | |
| II | 8:1 | 93 | 20,000 | infected | ++ | + | + | |
| | | | | normal | ++ | - | - | |
| | | | | Ha27-30 | +/- | +/- | +/- | |
| | | | | | | | | |
| II | 8:1 | 94 | 20,000 | infected | ++ | + | + | |
| | | | | normal | ++ | - | - | |
| | | | | Ha27-30 | + | + | + | |
| | | | | | | | | |
| III | 6:1 | 101 | 5,000 | infected | ++ | + | + | |
| | | | | normal | ++ | + | + | |
| | | | | Ha27-30 | ++ | ++ | ++ | |
| | | | | | | | | |
| III | 6:1 | 102 | 5,000 | infected | +++ | + | + | |
| | | | | normal | +++ | +/- | +/- | |
| | | | | Ha27-30 | ++ | ++ | ++ | |
| Vc | 5:1 | 97 | 160,000 | infected | +++ | +/- | +++ | + |
| | | | | normal | +++ | +/- | +/- | + |
| | | | | Ha27-30 | +++ | ++ | +++ | + |
| | | | | | | | | |
| Vc | 5:1 | 98 | 320,000 | infected | +++ | +/- | +++ | + |
| | | | | normal | +++ | +/- | +/- | + |
| | | | | Ha27-30 | +++ | +/- | +++ | + |

**Table II:**

| Results from ovine peptide sequences | | | | | | | | |
|---|---|---|---|---|---|---|---|---|
| | Pept/carrier ratio | Antibody number | Titre | Bovine Brain Material | DOT BLOT | | | West Blot |
| | | | | | Untrt | Prot K | Prot K + Gua | |
| II | 8:1 | 93 | 20,000 | infected | ++ | + | + | |
| | | | | normal | ++ | + | + | |
| | | | | Ha27-30 | +/- | +/- | +/- | |
| | | | | | | | | |
| II | 8:1 | 94 | 20,000 | infected | ++ | + | + | |
| | | | | normal | + | + | + | |
| | | | | Ha 27-30 | + | + | + | |
| III | 6:1 | 101 | 5,000 | infected | ++ | + | + | |
| | | | | normal | ++ | + | + | |
| | | | | Ha27-30 | ++ | ++ | ++ | |
| | | | | | | | | |
| III | 6:1 | 102 | 5,000 | infected | ++ | + | + | |
| | | | | normal | ++ | + | + | |
| | | | | Ha27-30 | ++ | ++ | ++ | |
| Vc | 5:1 | 97 | 160,000 | infected | +++ | + | ++ | |
| | | | | normal | ++ | + | + | |
| | | | | Ha27-30 | +++ | ++ | +++ | |
| | | | | | | | | |
| Vc | 5:1 | 98 | 320,000 | infected | +++ | + | ++ | |
| | | | | normal | ++ | +/- | +/- | |
| | | | | Ha27-30 | +++ | +/- | +++ | |

**Table III:**

| Results from ovine/bovine peptide sequences | | | | | | | | |
|---|---|---|---|---|---|---|---|---|
| | Pept/carrier ratio | Antibody number | Titre | Ovine Brain Material | DOT BLOT | | | West Blot |
| | | | | | Uₙₜᵣₜ | Prot | K Prot K + Gua | |
| Vb | 6:1 | 95 | 50,000 | infected | ++ | + | + | |
| | | | | normal | ++ | - | - | |
| | | | | Ha27-30 | ++ | ++ | ++ | |
| | | | | | | | | |
| Vb | 6:1 | 96 | 10,000 | infected | ++ | + | + | |
| | | | | normal | ++ | - | - | |
| | | | | Ha27-30 | ++ | ++ | ++ | |
| VIIIb | 12:1 | 103 | 3,000 | infected | ++ | + | + | |
| | | | | normal | ++ | + | + | |
| | | | | Ha27-30 | ++ | +/- | +/- | |
| | | | | | | | | |
| VIIIb | 12:1 | 104 | 3,000 | infected | + | + | + | |
| | | | | normal | + | + | + | |
| | | | | Ha27-30 | + | + | + | |

**Table IV:**

| Results from ovine/bovine peptide sequences | | | | | | | | |
|---|---|---|---|---|---|---|---|---|
| | Pept/carrier ratio | Antibody number | Titre | Bovine Brain Material | DOT BLOT | | | West Blot |
| | | | | | Untrt | Prot | KProt K + Gua | |
| Vb | 6:1 | 95 | 50,000 | infected | ++ | + | + | |
| | | | | normal | ++ | + | + | |
| | | | | Ha27-30 | ++ | ++ | ++ | |
| | | | | | | | | |
| Vb | 6:1 | 96 | 10,000 | infected | ++ | + | + | |
| | | | | normal | ++ | + | + | |
| | | | | Ha27-30 | ++ | ++ | ++ | |
| VIIIb | 12:1 | 103 | 3,000 | infected | ++ | + | + | |
| | | | | normal | ++ | + | + | |
| | | | | Ha27-30 | ++ | +/- | +/- | |
| | | | | | | | | |
| VIIIb | 12:1 | 104 | 3,000 | infected | + | + | + | |
| | | | | normal | ++ | +/- | +/- | |
| | | | | Ha27-30 | + | + | + | |

**Table V:**

| Results from bovine peptide sequences | | | | | | | | |
|---|---|---|---|---|---|---|---|---|
| | Pept/carrier ratio | Antibody number | Titre | Bovine Brain Material | DOT BLOT | | | West Blot |
| | | | | | Untrt | Prot K | Prot K + Gua | |
| BII | 9:1 | 105 | 100,000 | infected | +++ | + | + | |
| | | | | normal | +++ | + | + | |
| | | | | Ha27-30 | + | + | + | |
| BII | 9:1 | 106 | 100,000 | infected | +++ | + | + | |
| | | | | normal | +++ | + | + | |
| | | | | Ha27-30 | + | + | + | |
| BIII | 5:1 | 107 | 20,000 | infected | +++ | +/- | +/- | |
| | | | | normal | +++ | +/- | +/- | |
| | | | | Ha27-30 | + | + | + | |
| BIII | 5:1 | 108 | 10,000 | infected | +++ | +/- | +/- | |
| | | | | normal | +++ | +/- | +/- | |
| | | | | Ha27-30 | + | + | + | |

### SEQUENCE LISTING

### Number of Sequences 51

(1) Information for Seq. I.D. No: 1
   (i) Characterisation of sequence:
      (A) Length: 31 Amino acids
      (B) Type: Amino acid
      (D) Topology: Linear
   (ii) Type of molecule: Peptide
   (xi) Description of sequence: Seq. I.D. No: 1 
Met Lys His Val Ala Gly Ala Ala Ala Ala Gly Ala Val Val Gly Gly
1               5                   10                  15
Leu Gly Gly Tyr Met Leu Gly Ser Ala Met Ser Arg Pro Leu Ile
            20                  25                  30
(2) Information for Seq. I.D. No: 2
   (i) Characterisation of sequence:
      (A) Length: 31 Amino acids
      (B) Type: Amino acid
      (D) Topology: Linear
   (ii) Type of molecule: Peptide
   (xi) Description of sequence: Seq. I.D. No: 2 
Met Lys His Met Ala Gly Ala Ala Ala Ala Gly Ala Val Val Gly Gly
1               5                   10                  15
Leu Gly Gly Tyr Met Leu Gly Ser Ala Met Ser Arg Pro Ile Ile
            20                  25                  30
(3) Information for Seq. I.D. No: 3
   (i) Characterisation of sequence:
      (A) Length: 17 Amino acids
      (B) Type: Amino acid
      (D) Topology: Linear
   (ii) Type of molecule: Peptide
   (xi) Description of sequence: Seq. I.D. No: 3 
His Val Ala Gly Ala Ala Ala Ala Gly Ala Val Val Gly Gly Leu Gly
1               5                   10                  15
Gly
(4) Information for Seq. I.D. No: 4
   (i) Characterisation of sequence:
      (A) Length: 17 Amino acids
      (B) Type: Amino acid
      (D) Topology: Linear
   (ii) Type of molecule: Peptide
   (xi) Description of sequence: Seq. I.D. No: 4 
Gly Gly Leu Gly Gly Tyr Met Leu Gly Ser Ala Met Ser Arg Pro Leu
1               5                   10                  15
Ile
(5) Information for Seq. I.D. No: 5
   (i) Characterisation of sequence:
      (A) Length: 17 Amino acids
      (B) Type: Amino acid
      (D) Topology: Linear
   (ii) Type of molecule: Peptide
   (xi) Description of sequence: Seq. I.D. No: 5 
His Met Ala Gly Ala Ala Ala Ala Gly Ala Val Val Gly Gly Leu Gly
1               5                   10                  15
Gly
(6) Information for Seq. I.D. No: 6
   (i) Characterisation of sequence:
      (A) Length: 17 Amino acids
      (B) Type: Amino acid
      (D) Topology: Linear
   (ii) Type of molecule: Peptide
   (xi) Description of sequence: Seq. I.D. No: 6 
Gly Gly Leu Gly Gly Tyr Met Leu Gly Ser Ala Met Ser Arg Pro Ile
1               5                   10                  15
Ile
(7) Information for Seq. I.D. No: 7
   (i) Characterisation of sequence:
      (A) Length: 29 Amino acids
      (B) Type: Amino acid
      (D) Topology: Linear
   (ii) Type of molecule: Peptide
   (xi) Description of sequence: Seq. I.D. No: 7 
Ser Ala Met Ser Arg Pro Leu Ile His Phe Gly Ser Asp Tyr Glu Asp
1               5                   10                  15
Arg Tyr Tyr Arg Glu Asn Met His Arg Tyr Pro Asn Gln
            20                  25
(8) Information for Seq. I.D. No: 8
   (i) Characterisation of sequence:
      (A) Length: 29 Amino acids
      (B) Type: Amino acid
      (D) Topology: Linear
   (ii) Type of molecule: Peptide
   (xi) Description of sequence: Seq. I.D. No: 8 
Ser Ala Met Ser Arg Pro Leu Ile His Phe Gly Asn Asp Tyr Glu Asp
1               5                   10                  15
Arg Tyr Tyr Arg Glu Asn Met Tyr Arg Tyr Pro Asn Gln
            20                  25
(9) Information for Seq. I.D. No: 9
   (i) Characterisation of sequence:
      (A) Length: 29 Amino acids
      (B) Type: Amino acid
      (D) Topology: Linear
   (ii) Type of molecule: Peptide
   (xi) Description of sequence: Seq. I.D. No: 9 
Ser Ala Met Ser Arg Pro Ile Ile His Phe Gly Ser Asp Tyr Glu Asp
1               5                   10                  15
Arg Tyr Tyr Arg Glu Asn Met His Arg Tyr Pro Asn Gln
            20                  25
(10) Information for Seq. I.D. No: 10
   (i) Characterisation of sequence:
      (A) Length: 23 Amino acids
      (B) Type: Amino acid
      (D) Topology: Linear
   (ii) Type of molecule: Peptide
   (xi) Description of sequence: Seq. I.D. No: 10 
Ser Ala Met Ser Arg Pro Leu Ile His Phe Gly Ser Asp Tyr Glu Asp
1               5                   10                  15
Arg Tyr Tyr Arg Glu Asn Met
            20
(11) Information for Seq. I.D. No: 11
   (i) Characterisation of sequence:
      (A) Length: 23 Amino acids
      (B) Type: Amino acid
      (D) Topology: Linear
   (ii) Type of molecule: Peptide
   (xi) Description of sequence: Seq. I.D. No: 11 
Ser Ala Met Ser Arg Pro Leu Ile His Phe Gly Asn Asp Tyr Glu Asp
1               5                   10                  15
Arg Tyr Tyr Arg Glu Asn Met
            20
(12) Information for Seq. I.D. No: 12
   (i) Characterisation of sequence:
      (A) Length: 23 Amino acids
      (B) Type: Amino acid
      (D) Topology: Linear
   (ii) Type of molecule: Peptide
   (xi) Description of sequence: Seq. I.D. No: 12 
Ser Ala Met Ser Arg Pro Ile Ile His Phe Gly Ser Asp Tyr Glu Asp
1               5                   10                  15
Arg Tyr Tyr Arg Glu Asn Met
            20
(13) Information for Seq. I.D. No: 13
   (i) Characterisation of sequence:
      (A) Length: 29 Amino acids
      (B) Type: Amino acid
      (D) Topology: Linear
   (ii) Type of molecule: Peptide
   (xi) Description of sequence: Seq. I.D. No: 13 
Asn Met His Arg Tyr Pro Asn Gln Val Tyr Tyr Arg Pro Val Asp Gln
1               5                   10                  15
Tyr Ser Asn Gln Asn Asn Phe Val His Asp Cys Val Asn
            20                  25
(14) Information for Seq. I.D. No: 14
   (i) Characterisation of sequence:
      (A) Length: 29 Amino acids
      (B) Type: Amino acid
      (D) Topology: Linear
   (ii) Type of molecule: Peptide
   (xi) Description of sequence: Seq. I.D. No: 14 
Asn Met Tyr Arg Tyr Pro Asn Gln Val Tyr Tyr Arg Pro Val Asp Arg
1               5                   10                  15
Tyr Ser Asn Gln Asn Asn Phe Val His Asp Cys Val Asn
            20                  25
(15) Information for Seq. I.D. No: 15.
   (i) Characterisation of sequence:
      (A) Length: 29 Amino acids
      (B) Type: Amino acid
      (D) Topology: Linear
   (ii) Type or molecule: Peptide
   (xi) Description of sequence: Seq. I.D. No: 15 
Asn Met His Arg Tyr Pro Asn Gln Val Tyr Tyr Arg Pro Met Asp Glu
1               5                   10                  15
Tyr Ser Asn Gln Asn Asn Phe Val His Asp Cys Val Asn
            20                  25
(16) Information for Seq. I.D. No: 16
   (i) Characterisation of sequence:
      (A) Length: 26 Amino acids
      (B) Type: Amino acid
      (D) Topology: Linear
   (ii) Type of molecule: Peptide
   (xi) Description of sequence: Seq. I.D. No: 16 
Arg Tyr Pro Asn Gln Val Tyr Tyr Arg Pro Val Asp Gln Tyr Ser Asn
1               5                   10                  15
Gln Asn Asn Phe Val His Asp Cys Val Asn
            20                  25
(17) Information for Seq. I.D. No: 17
   (i) Characterisation of sequence:
      (A) Length: 26 Amino acids
      (B) Type: Amino acid
      (D) Topology: Linear
   (ii) Type of molecule: Peptide
   (xi) Description of sequence: Seq. I.D. No: 17 
Arg Tyr Pro Asn Gln Val Tyr Tyr Arg Pro Val Asp Arg Tyr Ser Asn
1               5                   10                  15
Gln Asn Asn Phe Val His Asp Cys Val Asn
            20                  25
(18) Information for Seq. I.D. No: 18
   (i) Characterisation of sequence:
      (A) Length: 26 Amino acids
      (B) Type: Amino acid
      (D) Topology: Linear
   (ii) Type of molecule: Peptide
   (xi) Description of sequence: Seq. I.D. No: 18 
Arg Tyr Pro Asn Gln Val Tyr Tyr Arg Pro Met Asp Glu Tyr Ser Asn
1               5                   10                  15
Gln Asn Asn Phe Val His Asp Cys Val Asn
            20                  25
(19) Information for Seq. I.D. No: 19
   (i) Characterisation of sequence:
      (A) Length: 29 Amino acids
      (B) Type: Amino acid
      (D) Topology: Linear
   (ii) Type of molecule: Peptide
   (xi) Description of sequence: Seq. I.D. No: 19 
Tyr Tyr Gln Arg Gly Ala Ser Val Ile Leu Phe Ser Ser Pro Pro Val
1               5                   10                  15
Ile Leu Leu Ile Ser Phe Leu Ile Phe Leu Ile Val Gly
            20                  25
(20) Information for Seq. I.D. No: 20
   (i) Characterisation of sequence:
      (A) Length: 29 Amino acids
      (B) Type: Amino acid
      (D) Topology: Linear
   (ii) Type of molecule: Peptide
   (xi) Description of sequence: Seq. I.D. No: 20 
Tyr Tyr Gln Arg Gly Ser Ser Met Val Leu Phe Ser Ser Pro Pro Val
1               5                   10                  15
Ile Leu Leu Ile Ser Phe Leu Ile Phe Leu Ile Val Gly
            20                  25
(21) Information for Seq. I.D. No: 21
   (i) Characterisation or sequence:
      (A) Length: 17 Amino acids
      (B) Type: Amino acid
      (D) Topology: Linear
   (ii) Type of molecule: Peptide
   (xi) Description of sequence: Seq. I.D. No: 21 
Gly Ala Ser Val Ile Leu Phe Ser Ser Pro Pro Val Ile Leu Leu Ile
1               5                   10                  15
Ser
(22) Information for Seq. I.D. No: 22
   (i) Characterisation of sequence:
      (A) Length: 17 Amino acids
      (B) Type: Amino acid
      (D) Topology: Linear
   (ii) Type of molecule: Peptide
   (xi) Description of sequence: Seq. I.D. No: 22 
Gly Ser Ser Met Val Leu Phe Ser Ser Pro Pro Val Ile Leu Leu Ile
1               5                   10                  15
Ser
(23) Information for Seq. I.D. No: 23
   (i) Characterisation of sequence:
      (A) Length: 31 Amino acids
      (B) Type: Amino acid
      (D) Topology: Linear
   (ii) Type of molecule: Peptide
   (xi) Description of sequence: Seq. I.D. No: 23 
Pro Gly Gly Gly Trp Asn Thr Gly Gly Ser Arg Tyr Pro Gly Gln Gly
1               5                   10                  15
Ser Pro Gly Gly Asn Arg Tyr Pro Pro Gln Gly Gly Gly Gly Trp
            20                  25                  30
(24) Information for Seq. I.D. No: 24
   (i) Characterisation of sequence:
      (A) Length: 16 Amino acids
      (B) Type: Amino acid
      (D) Topology: Linear
   (ii) Type of molecule: Peptide
   (xi) Description of sequence: Seq. I.D. No: 24 
Gly Gly Gly Trp Gly Gln Pro His Gly Gly Gly Trp Gly Gln Pro His
1               5                   10                  15
(25) Information for Seq. I.D. No: 25
   (i) Characterisation of sequence:
      (A) Length: 28 Amino acids
      (B) Type: Amino acid
      (D) Topology: Linear
   (ii) Type of molecule: Peptide
   (xi) Description of sequence: Seq. I.D. No: 25 
Gly Gly Gly Trp Gly Gln Gly Gly Thr His Gly Gln Trp Asn Lys Pro
1               5                   10                  15
Ser Lys Pro Lys Thr Asn Met Lys His Val Ala Gly
            20                  25
(26) Information for Seq. I.D. No: 26
   (i) Characterisation of sequence:
      (A) Length: 31 Amino acids
      (B) Type: Amino acid
      (D) Topology: Linear
   (ii) Type of molecule: Peptide
   (xi) Description of sequence: Seq. I.D. No: 26 
Pro Gly Gly Gly Trp Asn Thr Gly Gly Ser Arg Tyr Pro Gly Gln Gly
1               5                   10                  15
Ser Pro Gly Gly Asn Arg Tyr Pro Pro Gln Gly Gly Gly Gly Trp
            20                  25                  30
(27) Information for Seq. I.D. No: 27
   (i) Characterisation of sequence:
      (A) Length: 16 Amino acids
      (B) Type: Amino acid
      (D) Topology: Linear
   (ii) Type of molecule: Peptide
   (xi) Description of sequence: Seq. I.D. No: 27 
Gly Gly Gly Trp Gly Gln Pro His Gly Gly Gly Trp Gly Gln Pro His
1               5                   10                  15
(28) Information for Seq. I.D. No: 28
   (i) Characterisation of sequence:
      (A) Length: 28 Amino acids
      (B) Type: Amino acid
      (D) Topology: Linear
   (ii) Type of molecule: Peptide
   (xi) Description of sequence: Seq. I.D. No: 28 
Gly Gly Gly Trp Gly Gln Gly Gly Ser His Ser Gln Trp Asn Lys Pro
1               5                   10                  15
Ser L[?] Pro Lys Thr Asn Met Lys His Val Ala Gly
            20                  25
(29) Information for Seq. I.D. No: 29
   (i) Characterisation of sequence:
      (A) Length: 31 Amino acids
      (B) Type: Amino acid
      (D) Topology: Linear
   (ii) Type of molecule: Peptide
   (xi) Description of sequence: Seq. I.D. No: 29 
Pro Gly Gly Gly Trp Asn Thr Gly Gly Ser Arg Tyr Pro Gly Gln Gly
1               5                   10                  15
Ser Pro Gly Gly Asn Arg Tyr Pro Pro Gln Gly Gly Gly Gly Trp
            20                  25                  30
(30) Information for Seq. I.D. No: 30
   (i) Characterisation of sequence:
      (A) Length: 16 Amino acids
      (B) Type: Amino acid
      (D) Topology: Linear
   (ii) Type of molecule: Peptide
   (xi) Description of sequence: Seq. I.D. No: 30 
Gly Gly Gly Trp Gly Gln Pro His Gly Gly Gly Trp Gly Gln Pro His
1               5                   10                  15
(31) Information for Seq. I.D. No: 31
   (i) Characterisation of sequence:
      (A) Length: 29 Amino acids
      (B) Type: Amino acid
      (D) Topology: Linear
   (ii) Type of molecule: Peptide
   (xi) Description of sequence: Seq. I.D. No: 31 
Gly Gly Gly Trp Gly Gln Gly Gly Gly Thr His Ser Gln Trp Asn Lys
1               5                   10                  15
Pro Ser Lys Pro Lys Thr Asn Met Lys His Met Ala Gly
            20                  25
(32) Information for Seq. I.D. No: 32
   (i) Characterisation of sequence:
      (A) Length: 31 Amino acids
      (B) Type: Amino acid
      (D) Topology: Linear
   (ii) Type of molecule: Peptide
   (xi) Description of sequence: Seq. I.D. No: 32 
Asn Phe Val His Asp Cys Val Asn Ile Thr Val Lys Glu His Thr Val
1               5                   10                  15
Thr Thr Thr Thr Lys Gly Glu Asn Phe Thr Glu Thr Asp Ile Lys
            20                  25                  30
(33) Information for Seq. I.D. to: 33
   (i) Characterisation or sequence:
      (A) Length: 20 Amino acids
      (B) Type: Amino acid
      (D) Topology: Linear
   (ii) Type of molecule: Peptide
   (xi) Description of sequence: Seq. I.D. No: 33 
Met Cys Ile Thr Gln Tyr Gln Arg Glu Ser Gln Ala Tyr Tyr Gln Arg
1               5                   10                  15
Gly Ala Ser Val
            20
(34) Information for Seq. I.D. No: 34
   (i) Characterisation of sequence:
      (A) Length: 31 Amino acids
      (B) Type: Amino acid
      (D) Topology: Linear
   (ii) Type of molecule: Peptide
   (xi) Description of sequence: Seq. I.D. No: 34 
Asn Phe Val His Asp Cys Val Asn Ile Thr Val Lys Gln His Thr Val
1               5                   10                  15
Thr Thr Thr Thr Lys Gly Glu Asn Phe Thr Glu Thr Asp Ile Lys
            20                  25                  30
(35) Information for Seq. I.D. No: 35
   (i) Characterisation of sequence:
      (A) Length: 20 Amino acids
      (B) Type: Amino acid
      (D) Topology: Linear
   (ii) Type of molecule: Peptide
   (xi) Description of sequence: Seq. I.D. No: 35 
Met Cys Ile Thr Gln Tyr Gln Arg Glu Ser Gln Ala Tyr Tyr Gln Arg
1               5                   10                  15
Gly Ala Ser Val
            20
(36) Information for Seq. I.D. No: 36
   (i) Characterisation of sequence:
      (A) Length: 31 Amino acids
      (B) Type: Amino acid
      (D) Topology: Linear
   (ii) Type of molecule: Peptide
   (xi) Description of sequence: Seq. I.D. No: 36 
Asn Phe Val His Asp Cys Val Asn Ile Thr Ile Lys Gln His Thr Val
1               5                   10                  15
Thr Thr Thr Thr Lys Gly Glu Asn Phe Thr Glu Thr Asp Val Lys
            20                  25                  30
(37) Information for Seq. I.D. No: 37
   (i) Characterisation of sequence:
      (A) Length: 20 Amino acids
      (B) Type: Amino acid
      (D) Topology: Linear
   (ii) Type of molecule: Peptide
   (xi) Description of sequence: Seq. I.D. No: 37 
Met Cys Ile Thr Gln Tyr Glu Arg Glu Ser Gln Ala Tyr Tyr Gln Arg
1               5                   10                  15
Gly Ser Ser Met
            20
(38) Information for Seq. I.D. No: 38
   (i) Characterisation of sequence:
      (A) Length: 5 Amino acids
      (B) Type: Amino acid
      (D) Topology: Linear
   (ii) Type of molecule: Peptide
   (xi) Description of sequence: Seq. I.D. No: 38 
Gly Gly Gly Gly Gly
1               5
(39) Information for Seq. I.D. No: 39
   (i) Characterisation of sequence:
      (A) Length: 6 Amino acids
      (B) Type: Amino acid
      (D) Topology: Linear
   (ii) Type of molecule: Peptide
   (xi) Description of sequence: Seq. I.D. No: 39 
Gly Pro Gly Pro Gly Pro
1               5
(40) Information for Seq. I.D. No: 40
   (i) Characterisation cf sequence:
      (A) Length: 7 Amino acids
      (B) Type: Amino acid
      (D) Topology: Linear
   (ii) Type of molecule: Peptide
   (xi) Description of sequence: Seq. I.D. No: 40 
Gly Ser Ala Gly Ser Gly Ala
1               5
(41) Information for Seq. I.D. No: 41
   (i) Characterisation of sequence:
      (A) Length: 26 Amino acids
      (B) Type: Amino acid
      (D) Topology: Linear
   (ii) Type of molecule: Peptide
   (xi) Description of sequence: Seq. I.D. No: 41 
Ala Met Ser Arg Pro Leu Ile His Phe Gly Ser Asp Tyr Glu Asp Arg
1               5                   10                  15
Tyr Tyr Arg Glu Asn Met His Arg Gly Cys
            20                  25
(42) Information for Seq. I.D. No: 42
   (i) Characterisation of sequence:
      (A) Length: 21 Amino acids
      (B) Type: Amino acid
      (D) Topology: Linear
   (ii) Type of molecule: Peptide
   (xi) Description of sequence: Seq. I.D. No: 42 
Ser Ala Met Ser Arg Pro Leu Ile His Phe Gly Asn Asp Tyr Glu Asp
1               5                   10                  15
Arg Tyr Tyr Gly Cys
            20
(43) Information for Seq. I.D. No: 43
   (i) Characterisation of sequence:
      (A) Length: 21 Amino acids
      (B) Type: Amino acid
      (D) Topology: Linear
   (ii) Type of molecule: Peptide
   (xi) Description of sequence: Seq. I.D. No: 43 
Ser Ala Met Ser Arg Pro Leu Ile His Phe Gly Ser Asp Tyr Glu Asp
1               5                   10                  15
Arg Tyr Tyr Gly Cys
            20
(44) Information for Seq. I.D. No: 44
   (i) Characterisation of sequence:
      (A) Length: 27 Amino acids
      (B) Type: Amino acid
      (D) Topology: Linear
   (ii) Type of molecule: Peptide
   (xi) Description of sequence: Seq. I.D. No: 44 
Asn Met Tyr Arg Tyr Pro Asn Gln Val Tyr Tyr Arg Pro Val Asp Arg
1               5                   10                  15
Tyr Ser Asn Gln Asn Asn Phe Val His Gly Cys
            20                  25
(45) Information for Seq. I.D. No: 45
   (i) Characterisation of sequence:
      (A) Length: 27 Amino acids
      (B) Type: Amino acid
      (D) Topology: Linear
   (ii) Type of molecule: Peptide
   (xi) Description of sequence: Seq. I.D. No: 45 
Asn Met His Arg Tyr Pro Asn Gln Val Tyr Tyr Arg Pro Val Asp Gln
1               5                   10                  15
Tyr Ser Asn Gln Asn Asn Phe Val His Gly Cys
            20                  25
(46) Information for Seq. I.D. No: 46
   (i) Characterisation of sequence:
      (A) Length: 26 Amino acids
      (B) Type: Amino acid
      (D) Topology: Linear
   (ii) Type of molecule: Peptide
   (xi) Description of sequence: Seq. I.D. No: 46 
Gly Gln Pro His Gly Gly Gly Trp Gly Gln Pro His Gly Gly Gly Trp
1               5                   10                  15
Gly Gln Pro His Gly Gly Gly Trp Gly Cys
            20                  25
(47) Information for Seq. I.D. No: 47
   (i) Characterisation of sequence:
      (A) Length: 24 Amino acids
      (B) Type: Amino acid
      (D) Topology: Linear
   (ii) Type of molecule: Peptide
   (xi) Description of sequence: Seq. I.D. No: 47 
Gly Gln Gly Gly Ser His Ser Gln Trp Asn Lys Pro Ser Lys Pro Lys
1               5                   10                  15
Thr Asn Met Lys His Val Gly Cys
            20
(48) Information for Seq. I.D. No: 48
   (i) Characterisation of sequence:
      (A) Length: 15 Amino acids
      (B) Type: Amino acid
      (D) Topology: Linear
   (ii) Type of molecule: Peptide
   (xi) Description of sequence: Seq. I.D. No: 48 
Cys Ile Thr Gln Tyr Gln Arg Glu Ser Gln Ala Tyr Tyr Gln Arg
1               5                   10                  15
(49) Information for Seq. I.D. No: 49
   (i) Characterisation of sequence:
      (A) Length: 28 Amino acids
      (B) Type: Amino acid
      (D) Topology: Linear
   (ii) Type of molecule: Peptide
   (xi) Description of sequence: Seq. I.D. No: 49 
Gly Gly Trp Asn Thr Gly Gly Ser Arg Tyr Pro Gly Gln Gly Ser Pro
1               5                   10                  15
Gly Gly Asn Arg Tyr Pro Pro Gln Gly Gly Gly Cys
            20                  25
(50) Information for Seq. I.D. No: 50
   (i) Characterisation of sequence:
      (A) Length: 23 Amino acids
      (B) Type: Amino acid
      (D) Topology: Linear
   (ii) Type of molecule: Peptide
   (xi) Description of sequence: Seq. I.D. No: 50 
Val Asn Ile Thr Val Lys Gln His Thr Val Thr Thr Thr Thr Lys Gly
1               5                   10                  15
Glu Asn Phe Thr Glu Gly Cys
            20
(51) Information for Seq. I. D. No: 51
   (i) Characterisation of sequence:
      (A) Length: 29 Amino acids
      (B) Type: Amino acid
      (D) Topology: Linear
   (ii) Type of molecule: Peptide
   (xi) Description of sequence: Seq. I.D. No: 51

Lys His Met Ala Gly Ala Ala Ala Ala Gly Ala Val Val Gly Gly Leu
1               5                   10                  15
Gly Gly Tyr Met Leu Gly Ser Ala Met Ser Arg Gly Cys.
            20                  25

## Claims

1. A synthetic polypeptide having at least one antigenic site of a prion protein comprising a sequence according to general formulae Va, Vb and Vc: 
X-(Pro-Gly-Gly-R20)-Trp-Asn-Thr-Gly-Gly-Ser-Arg-Tyr-
Pro-Gly-Gln-Gly-Ser-Pro-Gly-Gly-Asn-Arg-Tyr-Pro-Pro-
Gln-Gly-(Gly-R21-R22-Trp)-Y    (Va);
 
X-(Gly-Gly-R21-R22-Trp)-Gly-Gln-Pro-His-Gly-Gly-Gly-
R23-Trp-(Gly-Gln-Pro-His)-Y (Vb);
 and 
X-(Gly-Gly-Gly-Trp)-Gly-Gln-Gly-Gly-R24-R25-His-R26-
Gln-Trp-Asn-Lys-Pro-R27-Lys-Pro-Lys-Thr-R28-R29-Lys
(-His-R30-Ala-Gly)-Y    (Vc)
 Wherein
R₂₀, R₂₁, R₂₃ and R₂₄ are each independently either Gly or absent;
R₂₂ is either Gly or Thr;
R₂₅ is either Thr or Ser;
R₂₆ is an amino acid residue selected from Gly, Ser and Asn;
R₂₇ and R₂₈ are each independently either Asn or Ser;
R₂₉ is an amino acid residue selected from Met, Leu and Phe;
R₃₀ is either Val or Met; one or more residues within brackets maybe present or absent with the proviso that if they are present they are attached to the rest of the peptide in sequence; and X and Y may each independently be absent or independently be one or more additional amino acid residues, with the proviso that when present neither X nor Y provide or form part of an antigenic property of the prion protein which, in the corresponding portion of sequence of a natural prion protein, is contiguous with the sequence to which X and Y are attached.

2. A synthetic polypeptide as claimed in claim 1 comprising a sequence selected from 
Seq. I.D. No: 23
X-(Pro-Gly-Gly-Gly)-Trp-Asn-Thr-Gly-Gly-Ser-Arg-Tyr-
Pro-Gly-Gln-Gly-Ser-Pro-Gly-Gly-Asn-Arg-Tyr-Pro-Pro-
Gln-Gly-(Gly-Gly-Gly-Trp)-Y;
 
Seq. I.D. No: 24
X-(Gly-Gly-Gly-Trp)-Gly-Gln-Pro-His-Gly-Gly-Gly-Trp-
(Gly-Gln-Pro-His)-Y;
 
Seq. I.D. No: 25
X-(Gly-Gly-Gly-Trp)-Gly-Gln-Gly-Gly-Thr-His-Gly-Gln-
Trp-Asn-Lys-Pro-Ser-Lys-Pro-Lys-Thr-Asn-Met-Lys
(-His-Val-Ala-Gly)-Y;
 
Seq. I.D. No: 26
X-(Pro-Gly-Gly-Gly)-Trp-Asn-Thr-Gly-Gly-Ser-Arg-Tyr-
Pro-Gly-Gln-Gly-Ser-Pro-Gly-Gly-Asn-Arg-Tyr-Pro-
Pro-Gln-Gly-(Gly-Gly-Gly-Trp)-Y;
 
Seq. I.D. No: 27
X-(Gly-Gly-Gly-Trp)-Gly-Gln-Pro-His-Gly-Gly-
Gly-Trp-(Gly-Gln-Pro-His)-Y;
 
Seq. I.D. No: 28
X-(Gly-Gly-Gly-Trp)-Gly-Gln-Gly-Gly-Ser-His-
Ser-Gln-Trp-Asn-Lys-Pro-Ser-Lys-Pro-Lys-Thr-
Asn-Met-Lys(-His-Val-Ala-Gly)-Y;
 
Seq. I.D. No: 29
X-(Pro-Gly-Gly-Gly)-Trp-Asn-Thr-Gly-Gly-Ser-Arg-Tyr-Pro-
Gly-Gln-Gly-Ser-Pro-Gly-Gly-Asn-Arg-Tyr-Pro-Pro-
Gln-Gly-(Gly-Gly-Gly-Trp)-Y;
 
Seq. I.D. No: 30
X-(Gly-Gly-Gly-Trp)-Gly-Gln-Pro-His-Gly-Gly-Gly-
Trp-(Gly-Gln-Pro-His)-Y;
 and

Seq. I.D. No: 31
X-(Gly-Gly-Gly-Trp)-Gly-Gln-Gly-Gly-Gly-Thr-His-Ser-
Gln-Trp-Asn-Lys-Pro-Ser-Lys-Pro-Lys-Thr-Asn-Met-Lys
(-His-Met-Ala-Gly)-Y.

3. A synthetic polypeptide as claimed in claim 1 selected from Seq. I.D. No: 49 
Gly-Gly-Trp-Asn-Thr-Gly-Gly-Ser-Arg-Tyr-
Pro-Gly-Gln-Gly-Ser-Pro-Gly-Gly-Asn-Arg-Tyr-Pro-
Pro-Gln-Gly-Gly-Gly-Cys
 
Seq. I.D. No: 46
Gly-Gln-Pro-His-Gly-Gly-Gly-Trp-Gly-Gln-Pro-His-Gly-Gly-
Gly-Trp-Gly-Gln-Pro-His-Gly-Gly-Gly-Trp-Gly-Cys; and
 and

Seq. I.D. No: 47
Gly-Gln-Gly-Gly-Ser-His-Ser-Gln-Trp-Asn-Lys-Pro-
Ser-Lys-Pro-Lys-Thr-Asn-Met-Lys-His-Val-Gly-Cys.

4. A synthetic polypeptide having at least one antigenic site of a prion protein comprising a sequence according to general formula (I) : 
X-(R1-Lys-His-R2)-Ala-Gly-Ala-Ala-Ala-R3-Gly-Ala-Val-
Val-Gly-Gly-Leu-Gly-Gly-Tyr-Met-Leu-Gly-Ser-Ala-Met-
Ser-(Arg-Pro-R4-R5)-Y
(I)
 wherein
R₁ is an amino acid residue selected from Met, Leu and Phe;
R₂ is either Met or Val;
R₃ is Ala or is absent;
R₄ and R₅ are independently an amino acid residue selected from Leu, Ile and Met; one or more residues within brackets maybe present or absent with the proviso that if they are present they are attached to the rest of the peptide in sequence; and X and Y may each independently be absent or independently be one or more additional amino acid residues, with the proviso that when present neither X nor Y provide or form part of an antigenic property of the prion protein which, in the corresponding portion of sequence of a natural prion protein, is contiguous with the sequence to which X and Y are attached.

5. A synthetic polypeptide as claimed in claim 4 comprising a sequence selected from Seq. I.D. No: 1 
X-(Met-Lys-His-Val)-Ala-Gly-Ala-Ala-Ala-Ala-Gly-Ala-
Val-Val-Gly-Gly-Leu-Gly-Gly-Tyr-Met-Leu-Gly-Ser-Ala-
Met-Ser-(Arg-Pro-Leu-Ile)-Y;
 and

Seq. I.D. No:2
X-(Met-Lys-His-Met)-Ala-Gly-Ala-Ala-Ala-Ala-Gly-Ala-
Val-Val-Gly-Gly-Leu-Gly-Gly-Tyr-Met-Leu-Gly-Ser-Ala-
Met-Ser-(Arg-Pro-Ile-Ile)-Y.

6. A synthetic polypeptide as claimed in claim 4 consisting of the sequence Seq. I.D. No: 51

Lys-His-Met-Ala-Gly-Ala-Ala-Ala-Ala-Gly-Ala-
Val-Val-Gly-Gly-Leu-Gly-Gly-Tyr-Met-Leu-Gly-Ser-Ala-
Met-Ser-Arg-Gly-Cys.

7. A significant sub-fragment of a sequence claimed in claim 4 preferably selected from 
i) X-(His-R2-Ala-Gly)-Ala-Ala-Ala-R3-Gly-Ala-Val-
Val-(Gly-Gly-Leu-Gly)-Y
 and; 
ii) X-(Gly-Gly-Leu-Gly)-Gly-Tyr-Met-Leu-Gly-Ser-
Ala-Met-Ser-(Arg-Pro-R4-R5)-Y
 wherein R₂, R₃, R₄, R₅, X and Y are as defined for formula I and one or more residues in brackets may be absent or present as in formula I.

8. A sub-fragment as claimed in claim 7 selected from 
Seq. I.D. No: 3
i) X-(His-Val-Ala-Gly)-Ala-Ala-Ala-Ala-Gly-Ala-
Val-Val-Gly-(Gly-Leu-Gly-Gly)-Y;
 
Seq. I.D. No: 4
ii) X-(Gly-Gly-Leu-Gly)-Gly-Tyr-Met-Leu-Gly-Ser-
Ala-Met-Ser-(Arg-Pro-Leu-Ile)-Y
 
Seq. I.D. No: 5
i) X-(His-Met-Ala-Gly)-Ala-Ala-Ala-Ala-Gly-Ala-
Val-Val-Gly-(Gly-Leu-Gly-Gly)-Y;
 and

Seq. I.D. No: 6
ii) X-(Gly-Gly-Leu-Gly)-Gly-Tyr-Met-Leu-Gly-Ser-
Ala-Met-Ser-(Arg-Pro-Ile-Ile)-Y.

9. A synthetic polypeptide having at least one antigenic site of a prion protein comprising a sequence according to general formula II: 
X-(Ser-Ala-Met-Ser)-Arg-Pro-R4-R5-His-Phe-Gly-R6-
Asp-R7-Glu-Asp-Arg-Tyr-Tyr-Arg-Glu-Asn-Met-R8-Arg-
(Tyr-Pro-Asn-Gln)-Y
(II)
 wherein
R₄ and R₅ are the same as in formula I ;
R₆ is either Asn or Ser;
R₇ is either Tyr or Trp;
R₈ is an amino acid residue selected from His, Tyr and Asn;
one or more residues within brackets maybe present or absent with the proviso that if they are present they are attached to the rest of the peptide in sequence; and X and Y may each independently be absent or independently be one or more additional amino acid residues, with the proviso that when present neither X nor Y provide or form part of an antigenic property of the prion protein which, in the corresponding portion of sequence of a natural prion protein, is contiguous with the sequence to which X and Y are attached.

10. A synthetic polypeptide as claimed in claim 9 comprising a sequence selected from 
Seq. I.D. No: 7
X-(Ser-Ala-Met-Ser)-Arg-Pro-Leu-Ile-His-Phe-Gly-Ser-
Asp-Tyr-Glu-Asp-Arg-Tyr-Tyr-Arg-Glu-Asn-Met-His-Arg-(Tyr-
Pro-Asn-Gln)-Y;
 
Seq. I.D. No: 8
X-(Ser-Ala-Met-Ser)-Arg-Pro-Leu-Ile-His-Phe-Gly-Asn-
Asp-Tyr-Glu-Asp-Arg-Tyr-Tyr-Arg-Glu-Asn-Met-Tyr-Arg-(Tyr-
Pro-Asn-Gln)-Y;
 and

Seq. I.D. No: 9
X-(Ser-Ala-Met-Ser)-Arg-Pro-Ile-Ile-His-Phe-Gly-Ser-
Asp-Tyr-Glu-Asp-Arg-Tyr-Tyr-Arg-Glu-Asn-Met-His-Arg-(Tyr-
Pro-Asn-Gln)-Y.

11. A significant sub-fragment of a sequence as claimed in claim 9 preferably comprising the sequence:- 
X-(Ser-Ala-Met-Ser)-Arg-Pro-R4-R5-His-Phe-Gly-R6-
Asp-R7-Glu-Asp-Arg-Tyr-Tyr-(Arg-Glu-Asn-Met)-Y
 wherein R₄ to R₇, X and Y are as defined in formula II and one or more residues in brackets may be present or absent.

12. A synthetic polypeptide as claimed in claim 11 selected from Seq. I.D. No: 42 
Ser-Ala-Met-Ser-Arg-Pro-Leu-Ile-His-Phe-Gly-
Asn-Asp-Tyr-Glu-Asp-Arg-Tyr-Tyr-Gly-Cys;
 and

Seq. I.D. No: 43
Ser-Ala-Met-Ser-Arg-Pro-Leu-Ile-His-Phe-Gly-
Ser-Asp-Tyr-Glu-Asp-Arg-Tyr-Tyr-Gly-Cys.

13. A sub-fragment as claimed in claim 11 selected from 
Seq. I.D. No: 10
X-(Ser-Ala-Met-Ser)-Arg-Pro-Leu-Ile-His-Phe-Gly-Ser-
Asp-Tyr-Glu-Asp-Arg-Tyr-Tyr-(Arg-Glu-Asn-Met)-Y;
 
Seq. I.D. No: 11
X-(Ser-Ala-Met-Ser)-Arg-Pro-Leu-Ile-His-Phe-Gly-Asn-
Asp-Tyr-Glu-Asp-Arg-Tyr-Tyr-(Arg-Glu-Asn-Met)-Y;
 and

Seq. I.D. No: 12
X-(Ser-Ala-Met-Ser)-Arg-Pro-Ile-Ile-His-Phe-Gly-Ser-
Asp-Tyr-Glu-Asp-Arg-Tyr-Tyr-(Arg-Glu-Asn-Met)-Y.

14. A synthetic polypeptide having at least one antigenic site of a prion protein comprising a sequence according to general formula III: 
X-(Asn-Met-R8-Arg)-Tyr-Pro-Asn-Gln-Val-Tyr-Tyr-Arg-
Pro-R9-Asp-R10-Tyr-R11-Asn-Gln-Asn-Asn-Phe-Val-His-
(Asp-Cys-Val-Asn)-Y
(III)
 wherein
R₈ is an amino acid residue selected from His, Tyr and Asn;
R₉ is Val or Met;
R₁₀ is an amino acid residue selected from Gln, Glu and Arg;
R₁₁ is Ser or Asn; one or more residues within brackets maybe present or absent with the proviso that if they are present they are attached to the rest of the peptide in sequence; and X and Y may each independently be absent or independently be one or more additional amino acid residues, with the proviso that when present neither X nor Y provide or form part of an antigenic property of the prion protein which, in the corresponding portion of sequence of a natural prion protein, is contiguous with the sequence to which X and Y are attached.

15. A synthetic polypeptide as claimed in claim 14 comprising a sequence selected from 
Seq. I.D. No: 13
X-(Asn-Met-His-Arg)-Tyr-Pro-Asn-Gln-Val-Tyr-Tyr-Arg-
Pro-Val-Asp-Gln-Tyr-Ser-Asn-Gln-Asn-Asn-Phe-Val-His-(Asp-
Cys-Val-Asn)-Y;
 
Seq. I.D. No: 14
X-(Asn-Met-Tyr-Arg)-Tyr-Pro-Asn-Gln-Val-Tyr-Tyr-Arg-
Pro-Val-Asp-Arg-Tyr-Ser-Asn-Gln-Asn-Asn-Phe-Val-His-(Asp-
Cys-Val-Asn)-Y;
 and

Seq. I.D. No: 15
X-(Asn-Met-His-Arg)-Tyr-Pro-Asn-Gln-Val-Tyr-Tyr-Arg-
Pro-Met-Asp-Glu-Tyr-Ser-Asn-Gln-Asn-Asn-Phe-Val-His-(Asp-
Cys-Val-Asn)-Y.

16. A synthetic polypeptide as claimed in claim 15 selected from Seq. I.D. No: 44 
Asn-Met-Tyr-Arg-Tyr-Pro-Asn-Gln-Val-Tyr-Tyr-Arg-Pro-Val-
Asp-Arg-Tyr-Ser-Asn-Gln-Asn-Asn-Phe-Val-His-Gly-Cys;
 and

Seq. I.D. No: 45
Asn-Met-His-Arg-Tyr-Pro-Asn-Gln-Val-Tyr-Tyr-Arg-Pro-Val-
Asp-Gln-Tyr-Ser-Asn-Gln-Asn-Asn-Phe-Val-His-Gly-Cys.

17. A significant sub-fragment of a sequence as claimed in claim 14 preferably comprising the sequence: 
X-(Arg-Tyr-Pro-Asn)-Gln-Val-Tyr-Tyr-Arg-Pro-R9-Asp-
R10-Tyr-R11-Asn-Gln-Asn-Asn-Phe-Val-His-
(Asp-Cys-Val-Asn)-Y.
 wherein R₉, R₁₀, R₁₁, X and Y are as defined in formula (III).

18. A sub-fragment as claimed in claim 17 selected from 
Seq. I.D. No: 16
X-(Arg-Tyr-Pro-Asn)-Gln-Val-Tyr-Tyr-Arg-Pro-Val-Asp-
Gln-Tyr-Ser-Asn-Gln-Asn-Asn-Phe-Val-His-
(Asp-Cys-Val-Asn)-Y;
 
Seq. I.D. No: 17
X-(Arg-Tyr-Pro-Asn)-Gln-Val-Tyr-Tyr-Arg-Pro-Val-Asp-
Arg-Tyr-Ser-Asn-Gln-Asn-Asn-Phe-Val-His-
(Asp-Cys-Val-Asn)-Y;
 and

Seq. I.D. No: 18
X-(Arg-Tyr-Pro-Asn)-Gln-Val-Tyr-Tyr-Arg-Pro-Met-Asp-Glu-
Tyr-Ser-Asn-Gln-Asn-Asn-Phe-Val-His-
(Asp-Cys-Val-Asn)-Y.

19. A synthetic polypeptide having at least one antigenic site of a prion protein comprising a sequence according to general formula IV: 
X-(Tyr-Tyr-R12-R13-Arg)-R14-R15-Ser-R16-R17-R18-Leu-Phe-Ser-
Ser-Pro-Pro-Val-Ile-Leu-Leu-Ile-Ser-Phe-Leu-Ile-Phe-
Leu-R19-Val-Gly-Y
(IV)
 wherein
R₁₂ is Asp or Gln;
R₁₃ is Gly or absent;
R₁₄ is Gly or Arg;
R₁₅ is Ala or Ser;
R₁₆ is Ser or absent;
R₁₇ is an amino acid residue selected from Ala, Thr, Met and Val;
R₁₈ is Val or Ile;
R₁₉ is Ile or Met; one or more residues within brackets may be present or absent with the proviso that if they are present they are attached to the rest of the peptide in sequence; and X and Y may each independently be absent or independently be one or more additional amino acid residues, with the proviso that when present neither X nor Y provide or form part of an antigenic property of the prion protein which, in the corresponding portion of sequence of a natural prion protein, is contiguous with the sequence to which X and Y are attached.

20. A synthetic polypeptide as claimed in claim 19 comprising a sequence selected from 
Seq. I.D. No: 19
X-(Tyr-Tyr-Gln-Arg)-Gly-Ala-Ser-Val-Ile-Leu-Phe-Ser-
Ser-Pro-Pro-Val-Ile-Leu-Leu-Ile-Ser-Phe-Leu-Ile-Phe-
Leu-Ile-Val-Gly-Y;
 and

Seq. I.D. No: 20
X-(Tyr-Tyr-Gln-Arg)-Gly-Ser-Ser-Met-Val-Leu-Phe-Ser-Ser-
Pro-Pro-Val-Ile-Leu-Leu-Ile-Ser-Phe-Leu-Ile-
Phe-Leu-Ile-Val-Gly-Y.

21. A significant sub-fragment of a sequence as claimed in claim 19 preferably comprising the sequence: 
X-(R14-R15-Ser-R16-R17)-R18-Leu-Phe-Ser-Ser-Pro-Pro-Val-
Ile-(Leu-Leu-Ile-Ser)-Y
 Wherein R₁₄ to R₁₈, X and Y are as defined in formula IV and one or more residues within brackets may be present or absent as in formula IV.

22. A sub-fragment as claimed in claim 21 selected from 
Seq. I.D. No: 21
X-(Gly-Ala-Ser-Val)-Ile-Leu-Phe-Ser-Ser-Pro-Pro-Val-
Ile-(Leu-Leu-Ile-Ser)-Y;
 and

Seq. I.D. No: 22
X-(Gly-Ser-Ser-Met)-Val-Leu-Phe-Ser-Ser-Pro-Pro-Val-
Ile-(Leu-Leu-Ile-Ser)-Y.

23. A synthetic polypeptide having at least one antigenic site of a prion protein comprising a sequence according to formula VI: 
X-(R31-R32-Trp-R33)-Trp-Leu-Gly-R34-R35-R36-Trp-R37
(Trp-Leu-Gly-R38)-Y
(VI)
 Wherein
R₃₁ and R₃₅ are each independently either Ala or Thr; R₃₂ and R₃₆ are each independently an amino acid residue selected from Ser, Pro and Thr;
R₃₃ and R₃₇ are each independently either Trp or Arg;
R₃₄ and R₃₈ are each independently an amino acid residue selected from Ala, Ser, Pro and Thr; one or more residues within brackets maybe present or absent with the proviso that if they are present they are attached to the rest of the peptide in sequence; and X and Y may each independently be absent or independently be one or more additional amino acid residues, with the proviso that when present neither X nor Y provide or form part of an antigenic property of the prion protein which, in the corresponding portion of sequence of a natural prion protein, is contiguous with the sequence to which X and Y are attached.

24. A synthetic polypeptide having at least one antigenic site of a prion protein comprising a sequence according to formula VII: 
X-(R39-R40-Met-R41)-Val-Ala-Gly-R42-R43-R44-Met-R45-
(Val-Ala-Gly-R46)-Y
(VII)
 Wherein
R₃₉ and R₄₃ are each independently either Ser or Asn; R₄₀ and R₄₄ are each independently an amino acid residue selected from Pro, Leu and His, R₄₁ and R₄₅ are each independently Val or Glu; R₄₂ and R₄₆ are each independently selected from Val, Ala, Asp and Gly; one or more residues within brackets maybe present or absent with the proviso that if they are present they are attached to the rest of the peptide in sequence; and
X and Y may each independently be absent or independently be one or more additional amino acid residues, with the proviso that when present neither X nor Y provide or form part of an antigenic property of the prion protein which, in the corresponding portion of sequence of a natural prion protein, is contiguous with the sequence to which X and Y are attached.

25. A synthetic polypeptide having at least one antigenic site of a prion protein comprising a sequence according to formula VIIIa or VIIIb: 
X-(Asn-Phe-Val-His)-Asp-Cys-Val-Asn-Ile-Thr-R47-Lys-
R48-His-Thr-Val-R49-Thr-Thr-Thr-Lys-Gly-Glu-Asn-
Phe-Thr-Glu-(Thr-Asp-R50-Lys)-Y
(VIIIa)
 
X-(Met-Cys-R51-Thr)-Gln-Tyr-R52-R53-Glu-Ser-Gln-Ala-
Tyr-Tyr-R54-R55-Arg-(R56-R57-Ser-R58-R59)-Y
(VIIIb)
 Wherein
R₄₇ is either Ile or Val;
R₄₈ and R₅₂ are each independently either Gln or Glu;
R₄₉ is either Val or Thr;
R₅₀ is either Val or Ile;
R₅₁ is an amino acid residue selected from Ile, Thr and Val;
R₅₂ is Gln or Glu;
R₅₃ is either Arg or Lys;
R₅₄ is either Asp or Gln;
R₅₅ is Gly or is absent;
R₅₆ is either Gly or Arg;
R₅₇ is either Ala or Ser;
R₅₈ is Ser or absent;
R₅₉ is an amino acid residue selected from Ala, Thr, Met and Val;
one or more residues within brackets maybe present or absent with the proviso that if they are present they are attached to the rest of the peptide in sequence; and X and Y may each independently be absent or independently be one or more additional amino acid residues, with the proviso that when present neither X nor Y provide or form part of an antigenic property of the prion protein which, in the corresponding portion of sequence of a natural prion protein, is contiguous with the sequence to which X and Y are attached.

26. A synthetic polypeptide as claimed in claim 25 comprising a sequence selected from: 
Seq I.D. No: 32
X-(Asn-Phe-Val-His)-Asp-Cys-Val-Asn-Ile-Thr-Val-Lys-
Glu-His-Thr-Val-Thr-Thr-Thr-Thr-Lys-Gly-Glu-Asn-
Phe-Thr-Glu-(Thr-Asp-Ile-Lys)-Y;
 
Seq. I.D. No: 33
X-(Met-Cys-Ile-Thr)-Gln-Tyr-Gln-Arg-Glu-Ser-Gln-Ala-
 Tyr-Tyr-Gln-Arg-(Gly-Ala-Ser-Val)-Y; 
Seq. I.D. No: 34
X-(Asn-Phe-Val-His)-Asp-Cys-Val-Asn-Ile-Thr-Val-Lys-
Gln-His-Thr-Val-Thr-Thr-Thr-Thr-Lys-Gly-Glu-Asn-
Phe-Thr-Glu-(Thr-Asp-Ile-Lys)-Y;
 
Seq. I.D. No: 35
X-(Met-Cys-Ile-Thr)-Gln-Tyr-Gln-Arg-Glu-Ser-Gln-Ala-
Tyr-Tyr-Gln-Arg-(Gly-Ala-Ser-Val)-Y;
 
Seq. I.D. No: 36
X-(Asn-Phe-Val-His)-Asp-Cys-Val-Asn-Ile-Thr-Ile-Lys-
Gln-His-Thr-Val-Thr-Thr-Thr-Thr-Lys-Gly-Glu-Asn-
Phe-Thr-Glu-(Thr-Asp-Val-Lys)-Y;
 and

Seq. I.D. No: 37
X-(Met-Cys-Ile-Thr)-Gln-Tyr-Glu-Arg-Glu-Ser-Gln-Ala-
Tyr-Tyr-Gln-Arg-(Gly-Ser-Ser-Met)-Y.

27. A subfragment of a synthetic polypeptide as claimed in claim 25 selected from Seq. I.D. No: 50 
Val-Asn-Ile-Thr-Val-Lys-Gln-His-Thr-Val-Thr-Thr-Thr-Thr-
Lys-Gly-Glu-Asn-Phe-Thr-Glu-Gly-Cys;
 and

Seq. I.D. No: 48
Cys-Ile-Thr-Gln-Tyr-Gln-Arg-Glu-Ser-Gln-Ala-Tyr-Tyr-Gln-
Arg.

28. A synthetic polypeptide of general formula (IX) :
[Lₐ-F]ₘ-[L_{b}-G]ₙ-L_{c} (IX)
wherein F and G may each independently be a polypeptide or sub-fragment according to any one of Formulae I to VIIIb, L is a linking sequence, a, b and c are each independently 0 or 1 and m and n are each positive numbers.

29. A synthetic polypeptide which comprises an antigenically significant subfragment and/or antigenically significant variant of the above-identified polypeptide sequences as claimed in any one of claims 1 to 27.

30. A synthetic polypeptide as claimed in any one of the preceding claims additionally comprising a T-cell epitope.

31. A synthetic polypeptide as claimed in any one of the preceding claims including a retro-inverso amino acid.

32. A synthetic polypeptide as claimed in any one of preceding claims linked to a carrier.

33. A DNA molecule coding for at least one synthetic polypeptide as claimed in any one of claims 1 to 30.

34. A vaccine comprising at least one polypeptide as claimed in any one of claims 1 to 31 effective to promote prophylaxis against encephalopathies.

35. A kit for detecting prion proteins or antibodies against prion proteins which comprises at least one synthetic polypeptide as claimed in any one of claims 1 to 31.

36. A pharmaceutical composition containing as active ingredient, at least one polypeptide or polypeptide-carrier conjugate as claimed in any one of claims 1 to 32 in association with one or more pharmaceutically acceptable adjuvants, carriers and/or excipients.

37. Use of a synthetic polypeptide as claimed in any one of claims 1 to 32 for the preparation of a medicament for the therapeutic or prophylactic treatment of mammalian encephalopathies and/or blocking the cellular binding or aggregation of the prion proteins.

38. Use of a polypeptide as claimed in any one of claims 1 to 32, either in isolation or in combination with other agents, for the manufacture of a composition for therapy or prophylaxis of mammalian encephalopathies and/or of stimulating the mammalian immune system and/or of blocking the cellular binding or aggregation of the prion proteins.

39. A method of detecting prion protein or antibodies against prion protein or antigen binding fragments thereof, which comprises incubating a sample with at least one polypeptide as claimed in any one of claims 1 to 32.

40. A method of discriminating between PrP^{C} and PrP^{SC} in which a sample is contacted with a substance selected from peptide sequences as claimed in any one of claims 1 to 32 preferably those relating to regions A, B and C, and significant sub-fragments thereof, antibodies raised against said sequences and sub-fragments and the presence or absence of PrP^{SC} is determined.

41. An antibody or antigen binding fragment thereof which specifically binds to a synthetic polypeptide as claimed in any one of claims 1 to 31.

42. A kit for detecting prion proteins or antibodies against prion proteins which contains an antibody or antigen binding fragment thereof, as claimed in claim 41.

43. A pharmaceutical composition comprising, as active ingredient, an antibody or antigen binding fragment as claimed in claim 41 in association with one or more pharmaceutically acceptable, carriers and/or excipients.

44. Use of an antibody or antigen binding fragment as claimed in claim 41 for the manufacture of a composition for therapy or prophylaxis of mammalian encephalopathies.

45. A method detecting prion proteins or antibodies against prion proteins which comprises incubating a sample with an antibody or antigen binding fragment as claimed in claim 41.

46. An anti-idiotypic antibody raised against an antibody or antigen binding fragment as claimed in claim 41.

47. A process for the manufacture of a synthetic polypeptide having at least one antigenic site of a prion protein, the process comprising the steps of coupling the residues using chemical, biological or recombinant techniques known per se and isolating the polypeptide as defined in any one of claims 1 to 31.

48. A process for the manufacture of an antibody which specifically binds to a synthetic polypeptide having at least one antigenic site of a prion protein, the process comprising immunising a non-human mammal with said polypeptide and isolating the antibody as defined in claim 41.

## Patentansprüche

1. Synthetisches Polypeptid mit mindestens einer antigenen Stelle eines Prionproteins, umfassend eine Sequenz gemäß den allgemeinen Formeln Va, Vb und Vc: 
X-(Pro-Gly-Gly-R20)-Trp-Asn-Thr-Gly-Gly-Ser-Arg-Tyr-
Pro-Gly-Gln-Gly-Ser-Pro-Gly-Gly-Asn-Arg-Tyr-Pro-Pro-
Gln-Gly-(Gly-R21-R22-Trp)-Y (Va);
 
X-(Gly-Gly-R21- R22-Trp)-Gly-Gln-Pro-His-Gly-Gly-Gly-
R23-Trp-(Gly-Gln-Pro-His)-Y (Vb);
 und 
X-(Gly-Gly-Gly-Trp)-Gly-Gln-Gly-Gly-R24-R25-His-R26-
Gln-Trp-Asn-Lys-Pro-R27-Lys-Pro-Lys-Thr-R28-R29-Lys
(-His-R30-Ala-Gly)-Y (Vc),
 wobei
R₂₀, R₂₁, R₂₃ und R₂₄ jeweils unabhängig voneinander entweder Gly sind oder fehlen;
R₂₂ entweder Gly oder Thr ist;
R₂₅ entweder Thr oder Ser ist;
R₂₆ ein aus Gly, Ser und Asn ausgewählter Aminosäurerest ist;
R₂₇ und R₂₈ jeweils unabhängig voneinander entweder Asn oder Ser sind;
R₂₉ ein aus Met, Leu und Phe ausgewählter Aminosäurerest ist;
R₃₀ entweder Val oder Met ist; wobei ein oder mehrere Reste in Klammem vorhanden sein oder fehlen können, mit der Maßgabe, daß sie - wenn sie vorhanden sind - der Reihe nach an das restliche Pepid gebunden sind, und X und Y jeweils unabhängig voneinander fehlen können oder unabhängig voneinander ein oder mehrere weitere Aminosäurereste sein können, mit der Maßgabe, daß - wenn vorhanden - weder X noch Y einen Teil einer Antigeneigenschaft des Prionproteins bereitstellen oder bilden, der in dem entsprechenden Sequenzabschnitt eines natürlichen Prionproteins an die Sequenz grenzt, an die X und Y gebunden sind.

2. Synthetisches Polypeptid nach Anspruch 1, umfassend eine Sequenz, ausgewählt aus: 
Seq.-ID.-Nr. 23
X-(Pro-Gly-Gly-Gly)-Trp-Asn-Thr-Gly-Gly-Ser-Arg-Tyr-
Pro-Gly-Gln-Gly-Ser-Pro-Gly-Gly-Asn-Arg-Tyr-Pro-Pro-
Gln-Gly-(Gly-Gly-Gly-Trp)-Y;
 
Seq.-ID.-Nr. 24
X-(Gly-Gly-Gly-Trp)-Gly-Gln-Pro-His-Gly-Gly-Gly-Trp-
(Gly-Gln-Pro-His)-Y;
 
Seq.-ID.-Nr. 25
X-(Gly-Gly-Gly-Trp)-Gly-Gln-Gly-Gly-Thr-His-Gly-Gln-
Trp-Asn-Lys-Pro-Ser-Lys-Pro-Lys-Thr-Asn-Met-Lys
(-His-Val-Ala-Gly)-Y;
 
Seq.-ID.-Nr. 26
X-(Pro-Gly-Gly-Gly)-Trp-Asn-Thr-Gly-Gly-Ser-Arg-Tyr-
Pro-Gly-Gln-Gly-Ser-Pro-Gly-Gly-Asn-Arg-Tyr-Pro-
Pro-Gln-Gly-(Gly-Gly-Gly-Trp)-Y;
 
Seq.-ID.-Nr. 27
X-(Gly-Gly-Gly-Trp)-Gly-Gln-Pro-His-Gly-Gly-
Gly-Trp-(Gly-Gln-Pro-His)-Y;
 
Seq.-ID.-Nr. 28
X-(Gly-Gly-Gly-Trp)-Gly-Gln-Gly-Gly-Ser-His-
Ser-Gln-Trp-Asn-Lys-Pro-Ser-Lys-Pro-Lys-Thr-
Asn-Met-Lys-(His-Val-Ala-Gly)-Y;
 
Seq.-ID.-Nr. 29
X-(Pro-Gly-Gly-Gly)-Trp-Asn-Thr-Gly-Gly-Ser-Arg-Tyr-Pro-
Gly-Gln-Gly-Ser-Pro-Gly-Gly-Asn-Arg-Tyr-Pro-Pro-
Gln-Gly-(Gly-Gly-Gly-Trp)-Y;
 
Seq.-ID.-Nr. 30
X-(Gly-Gly-Gly-Trp)-Gly-Gln-Pro-His-Gly-Gly-Gly-
Trp-(Gly-Gln-Pro-His)-Y;
 und

Seq.-ID.-Nr. 31
X-(Gly-Gly-Gly-Trp)-Gly-Gln-Gly-Gly-Gly-Thr-His-Ser-
Gln-Trp-Asn-Lys-Pro-Ser-Lys-Pro-Lys-Thr-Asn-Met-Lys-
(His-Met-Ala-Gly)-Y.

3. Synthetisches Polypeptid nach Anspruch 1, ausgewählt aus Seq.-ID.-Nr. 49 
Gly-Gly-Trp-Asn-Thr-Gly-Gly-Ser-Arg-Tyr-
Pro-Gly-Gln-Gly-Ser-Pro-Gly-Gly-Asn-Arg-Tyr-Pro-
Pro-Gln-Gly-Gly-Gly-Cys;
 
Seq.-ID.-Nr. 46
Gly-Gln-Pro-His-Gly-Gly-Gly-Trp-Gly-Gln-Pro-His-Gly-Gly-
Gly-Trp-Gly-Gln-Pro-His-Gly-Gly-Gly-Trp-Gly-Cys;
 und

Seq.-ID.-Nr. 47
Gly-Gln-Gly-Gly-Ser-His-Ser-Gln-Trp-Asn-Lys-Pro-
Ser-Lys-Pro-Lys-Thr-Asn-Met-Lys-His-Val-Gly-Cys.

4. Synthetisches Polypeptid mit mindestens einer antigenen Stelle eines Prionproteins, umfassend eine Sequenz gemäß der allgemeinen Formel (I): 
X-(R1-Lys-His-R2)-Ala-Gly-Ala-Ala-Ala-R3-Gly-Ala-Val-
Val-Gly-Gly-Leu-Gly-Gly-Tyr-Met-Leu-Gly-Ser-Ala-Met-
Ser-(Arg-Pro-R4-R5)-Y
(I),
 wobei
R₁ ein aus Met, Leu und Phe ausgewählter Aminosäurerest ist;
R₂ entweder Met oder Val ist;
R₃ Ala ist oder fehlt;
R₄ und R₅ unabhängig voneinander ein aus Leu, Ile und Met ausgewählter Aminosäurerest sind; wobei ein oder mehrere Reste in Klammern vorhanden sein oder fehlen können, mit der Maßgabe, daß sie - wenn sie vorhanden sind - der Reihe nach an das restliche Pepid gebunden sind, und X und Y jeweils unabhängig voneinander fehlen können oder unabhängig voneinander ein oder mehrere weitere Aminosäurereste sein können, mit der Maßgabe, daß - wenn vorhanden - weder X noch Y einen Teil einer Antigeneigenschaft des Prionproteins bereitstellen oder bilden, der in dem entsprechenden Sequenzabschnitt eines natürlichen Prionproteins an die Sequenz grenzt, an die X und Y gebunden sind.

5. Synthetisches Polypeptid nach Anspruch 4, umfassend eine Sequenz, ausgewählt aus 
Seq.-ID.-Nr. 1
X-(Met-Lys-His-Val)-Ala-Gly-Ala-Ala-Ala-Ala-Gly-Ala-
Val-Val-Gly-Gly-Leu-Gly-Gly-Tyr-Met-Leu-Gly-Ser-Ala-
Met-Ser-(Arg-Pro-Leu-Ile)-Y;
 und

Seq.-ID.-Nr. 2
X-(Met-Lys-His-Met)-Ala-Gly-Ala-Ala-Ala-Ala-Gly-Ala-
Val-Val-Gly-Gly-Leu-Gly-Gly-Tyr-Met-Leu-Gly-Ser-Ala-
Met-Ser-(Arg-Pro-Ile-Ile)-Y.

6. Synthetisches Polypeptid nach Anspruch 4, bestehend aus der Sequenz

Seq.-ID.-Nr. 51
Lys-His-Met-Ala-Gly-Ala-Ala-Ala-Ala-Gly-Ala-
Val-Val-Gly-Gly-Leu-Gly-Gly-Tyr-Met-Leu-Gly-Ser-Ala-
Met-Ser-Arg-Gly-Cys.

7. Signifikantes Subfragment einer Sequenz nach Anspruch 4, das vorzugsweise ausgewählt ist aus: 
i) X-(His-R2-Ala-Gly)-Ala-Ala-Ala-R3-Gly-Ala-Val-
Val-(Gly-Gly-Leu-Gly)-Y;
 und 
ii) X-(Gly-Gly-Leu-Gly)-Gly-Tyr-Met-Leu-Gly-Ser-
Ala-Met-Ser-(Arg-Pro-R4-R5)-Y,
 wobei R₂, R₃, R₄, R₅, X und Y wie für Formel I definiert sind, und ein oder mehrere Reste in Klammern wie in Formel I fehlen oder vorhanden sein können.

8. Subfragment nach Anspruch 7, ausgewählt aus 
Seq.-ID.-Nr. 3
i) X-(His-Val-Ala-Gly)-Ala-Ala-Ala-Ala-Gly-Ala-
Val-Val-Gly-(Gly-Leu-Gly-Gly)-Y;
 
Seq.-ID.-Nr. 4
ii) X-(Gly-Gly-Leu-Gly)-Gly-Tyr-Met-Leu-Gly-Ser-
Ala-Met-Ser-(Arg-Pro-Leu-Ile)-Y;
 
Seq.-ID.-Nr. 5
i) X-(His-Met-Ala-Gly)-Ala-Ala-Ala-Ala-Gly-Ala-
Val-Val-Gly-(Gly-Leu-Gly-Gly)-Y;
 und

Seq.-ID.-Nr. 6
ii) X-(Gly-Gly-Leu-Gly)-Gly-Tyr-Met-Leu-Gly-Ser-
Ala-Met-Ser-(Arg-Pro-Ile-Ile)-Y.

9. Synthetisches Polypeptid mit mindestens einer antigenen Stelle eines Prionproteins, umfassend eine Sequenz gemäß der allgemeinen Formel II: 
X-(Ser-Ala-Met-Ser)-Arg-Pro-R4-R5-His-Phe-Gly-R6-
Asp-R7-Glu-Asp-Arg-Tyr-Tyr-Arg-Glu-Asn-Met-R8-Arg-
(Tyr-Pro-Asn-Gln)-Y
(II),
 wobei
R₄ und R₅ die gleiche Bedeutung haben wie in Formel I;
R₆ entweder Asn oder Ser ist;
R₇ entweder Tyr oder Trp ist;
R₈ ein Aminosäurerest ist, ausgewählt aus His, Tyr und Asn;
wobei ein oder mehrere Reste in Klammern vorhanden sein oder fehlen können, mit der Maßgabe, daß sie -wenn sie vorhanden sind - der Reihe nach an das restliche Peptid gebunden sind, und
X und Y jeweils unabhängig voneinander fehlen können oder unabhängig voneinander ein oder mehrere zusätzliche Aminosäurereste sein können, mit der Maßgabe, daß - wenn vorhanden - weder X noch Y einen Teil einer Antigeneigenschaft des Prionproteins bereitstellen oder bilden, der in dem entsprechenden Sequenzabschnitt eines natürlichen Prionproteins an die Sequenz grenzt, an die X und Y gebunden sind.

10. Synthetisches Polypeptid nach Anspruch 9, umfassend eine Sequenz, ausgewählt aus 
Seq.-ID.-Nr. 7
X-(Ser-Ala-Met-Ser)-Arg-Pro-Leu-Ile-His-Phe-Gly-Ser-
Asp-Tyr-Glu-Asp-Arg-Tyr-Tyr-Arg-Glu-Asn-Met-His-Arg-(Tyr-
Pro-Asn-Gln)-Y;
 
Seq.-ID.-Nr. 8
X-(Ser-Ala-Met-Ser)-Arg-Pro-Leu-Ile-His-Phe-Gly-Asn-
Asp-Tyr-Glu-Asp-Arg-Tyr-Tyr-Arg-Glu-Asn-Met-Tyr-Arg-(Tyr-
Pro-Asn-Gln)-Y;
 und

Seq.-ID.-Nr. 9
X-(Ser-Ala-Met-Ser)-Arg-Pro-Ile-Ile-His-Phe-Gly-Ser-
Asp-Tyr-Glu-Asp-Arg-Tyr-Tyr-Arg-Glu-Asn-Met-His-Arg-(Tyr-
Pro-Asn-Gln)-Y.

11. Signifikantes Subfragment einer Sequenz nach Anspruch 9, das vorzugsweise die Sequenz umfaßt:- 
X-(Ser-Ala-Met-Ser)-Arg-Pro-R4-R5-His-Phe-Gly-R6-
Asp-R7-Glu-Asp-Arg-Tyr-Tyr-(Arg-Glu-Asn-Met)-Y,
 wobei R₄ bis R₇, X und Y wie für Formel II definiert sind, und ein oder mehrere Reste in Klammern vorhanden sein oder fehlen können.

12. Synthetisches Polypeptid nach Anspruch 11, ausgewählt aus 
Seq.-ID.-Nr. 42
Ser-Ala-Met-Ser-Arg-Pro-Leu-Ile-His-Phe-Gly-
Asn-Asp-Tyr-Glu-Asp-Arg-Tyr-Tyr-Gly-Cys;
 und

Seq.-ID.-Nr. 43
Ser-Ala-Met-Ser-Arg-Pro-Leu-Ile-His-Phe-Gly-
Ser-Asp-Tyr-Glu-Asp-Arg-Tyr-Tyr-Gly-Cys.

13. Subfragment nach Anspruch 11, ausgewählt aus: 
Seq.-ID.-Nr. 10
X-(Ser-Ala-Met-Ser)-Arg-Pro-Leu-Ile-His-Phe-Gly-Ser-
Asp-Tyr-Glu-Asp-Arg-Tyr-Tyr-(Arg-Glu-Asn-Met)-Y;
 
Seq.-ID.-Nr. 11
X-(Ser-Ala-Met-Ser)-Arg-Pro-Leu-Ile-His-Phe-Gly-Asn-
Asp-Tyr-Glu-Asp-Arg-Tyr-Tyr-(Arg-Glu-Asn-Met)-Y;
 und

Seq.-ID.-Nr. 12
X-(Ser-Ala-Met-Ser)-Arg-Pro-Ile-Ile-His-Phe-Gly-Ser-
Asp-Tyr-Glu-Asp-Arg-Tyr-Tyr-(Arg-Glu-Asn-Met)-Y.

14. Synthetisches Polypeptid mit mindestens einer antigenen Stelle eines Prionproteins, umfassend eine Sequenz gemäß der allgemeinen Formel III: 
X-(Asn-Met-R8-Arg)-Tyr-Pro-Asn-Gln-Val-Tyr-Tyr-Arg-
Pro-R9-Asp-R10-Tyr-R11-Asn-Gln-Asn-Asn-Phe-Val-His-
(Asp-Cys-Val-Asn)-Y
(III),
 wobei
R₈ ein aus His, Tyr und Asn ausgewählter Aminosäurerest ist,
R₉ Val oder Met ist;
R₁₀ ein aus Gln, Glu und Arg ausgewählter Aminosäurerest ist;
R₁₁ Ser oder Asn ist; wobei ein oder mehrere Reste in Klammern vorhanden sein oder fehlen können, mit der Maßgabe daß sie - wenn sie vorhanden sind - der Reihe nach an das restliche Peptid gebunden sind, und X und Y jeweils unabhängig voneinander fehlen können oder unabhängig voneinander ein oder mehrere zusätzliche Aminosäurereste sein können, mit der Maßgabe, daß - wenn vorhanden - weder X noch Y einen Teil einer Antigeneigenschaft des Prionproteins bereitstellen oder bilden, der in dem entsprechenden Sequenzabschnitt eines natürlichen Prionproteins an die Sequenz grenzt, an die X und Y gebunden sind.

15. Synthetisches Polypeptid nach Anspruch 14, umfassend eine Sequenz ausgewählt aus 
Seq.-ID.-Nr. 13
X-(Asn-Met-His-Arg)-Tyr-Pro-Asn-Gln-Val-Tyr-Tyr-Arg-
Pro-Val-Asp-Gln-Tyr-Ser-Asn-Gln-Asn-Asn-Phe-Val-His-(Asp-
Cys-Val-Asn)-Y;
 
Seq.-ID.-Nr. 14
X-(Asn-Met-Tyr-Arg)-Tyr-Pro-Asn-Gln-Val-Tyr-Tyr-Arg-
Pro-Val-Asp-Arg-Tyr-Ser-Asn-Gln-Asn-Asn-Phe-Val-His-(Asp-
Cys-Val-Asn)-Y;
 und

Seq.-ID.-Nr. 15
X-(Asn-Met-His-Arg)-Tyr-Pro-Asn-Gln-Val-Tyr-Tyr-Arg-
Pro-Met-Asp-Glu-Tyr-Ser-Asn-Gln-Asn-Asn-Phe-Val-His-(Asp-
Cys-Val-Asn)-Y.

16. Synthetisches Polypeptid nach Anspruch 15, ausgewählt aus 
Seq.-ID.-Nr. 44
Asn-Met-Tyr-Arg-Tyr-Pro-Asn-Gln-Val-Tyr-Tyr-Arg-Pro-Val-
Asp-Arg-Tyr-Ser-Asn-Gln-Asn-Asn-Phe-Val-His-Gly-Cys;
 und

Seq.-ID.-Nr. 45
Asn-Met-His-Arg-Tyr-Pro-Asn-Gln-Val-Tyr-Tyr-Arg-Pro-Val-
Asp-Gln-Tyr-Ser-Asn-Gln-Asn-Asn-Phe-Val-His-Gly-Cys.

17. Signifikantes Subfragment einer Sequenz nach Anspruch 14, das vorzugsweise die Sequenz umfaßt: 
X-(Arg-Tyr-Pro-Asn)-Gln-Val-Tyr-Tyr-Arg-Pro-R₉-Asp-
R₁₀-Tyr-R₁₁-Asn-Gln-Asn-Asn-Phe-Val-His-
(Asp-Cys-Val-Asn)-Y,
 wobei R₉, R₁₀, R₁₁, X und Y wie für Formel (III) definiert sind.

18. Subfragment nach Anspruch 17, ausgewählt aus 
Seq.-ID.-Nr. 16
X-(Arg-Tyr-Pro-Asn)-Gln-Val-Tyr-Tyr-Arg-Pro-Val-Asp-
Gln-Tyr-Ser-Asn-Gln-Asn-Asn-Phe-Val-His-
(Asp-Cys-Val-Asn)-Y;
 
Seq.-ID.-Nr. 17
X-(Arg-Tyr-Pro-Asn)-Gln-Val-Tyr-Tyr-Arg-Pro-Val-Asp-
Arg-Tyr-Ser-Asn-Gln-Asn-Asn-Phe-Val-His-
(Asp-Cys-Val-Asn)-Y;
 und

Seq.-ID.-Nr. 18
X-(Arg-Tyr-Pro-Asn)-Gln-Val-Tyr-Tyr-Arg-Pro-Met-Asp-Glu-
Tyr-Ser-Asn-Gln-Asn-Asn-Phe-Val-His-
(Asp-Cys-Val-Asn)-Y.

19. Synthetisches Polypeptid mit mindestens einer antigenen Stelle eines Prionproteins, umfassend eine Sequenz gemäß der allgemeinen Formel IV: 
X-(Tyr-Tyr-R₁₂-R₁₃-Arg)-R₁₄-R₁₅-Ser-R₁₆-R₁₇-R₁₈-Leu-Phe-Ser-
Ser-Pro-Pro-Val-Ile-Leu-Leu-Ile-Ser-Phe-Leu-Ile-Phe-
Leu-R₁₉-Val-Gly-Y
(IV),
 wobei
R₁₂ Asp oder Gln ist;
R₁₃ Gly ist oder fehlt;
R₁₄ Gly oder Arg ist;
R₁₅ Ala oder Ser ist;
R₁₆ Ser ist oder fehlt;
R₁₇ ein aus Ala, Thr, Met und Val ausgewählter Aminosäurerest ist;
R₁₈ Val oder Ile ist;
R₁₉ Ile oder Met ist; wobei ein oder mehrere Reste in Klammern vorhanden sein oder fehlen können, mit der Maßgabe, daß sie - wenn sie vorhanden sind - der Reihe nach an das restliche Peptid gebunden sind, und X und Y jeweils unabhängig voneinander fehlen können oder unabhängig voneinander ein oder mehrere zusätzliche Aminosäurereste sein können, mit der Maßgabe, daß - wenn vorhanden - weder X noch Y einen Teil einer Antigeneigenschaft des Prionproteins bereitstellen oder bilden, der im entsprechenden Sequenzabschnitt eines natürlichen Prionproteins an die Sequenz grenzt, an die X und Y gebunden sind.

20. Synthetisches Polypeptid nach Anspruch 19, umfassend eine Sequenz, ausgewählt aus 
Seq.-ID.-Nr. 19
X-(Tyr-Tyr-Gln-Arg)-Gly-Ala-Ser-Val-Ile-Leu-Phe-Ser-
Ser-Pro-Pro-Val-Ile-Leu-Leu-Ile-Ser-Phe-Leu-Ile-Phe-
Leu-Ile-Val-Gly-Y;
 und

Seq.-ID.-Nr. 20
X-(Tyr-Tyr-Gln-Arg)-Gly-Ser-Ser-Met-Val-Leu-Phe-Ser-Ser-
Pro-Pro-Val-Ile-Leu-Leu-Ile-Ser-Phe-Leu-Ile-
Phe-Leu-Ile-Val-Gly-Y.

21. Signifikantes Subfragment einer Sequenz nach Anspruch 19, das vorzugsweise die Sequenz umfaßt: 
X-(R14-R15-Ser-R16-R17)-R18-Leu-Phe-Ser-Ser-Pro-Pro-Val-
Ile-(Leu-Leu-Ile-Ser)-Y,
 wobei R₁₄ bis R₁₈, X und Y wie für Formel IV definiert sind, und ein oder mehrere Reste in Klammern wie in Formel IV vorhanden sein oder fehlen können.

22. Subfragment nach Anspruch 21, ausgewählt aus 
Seq.-ID.-Nr. 21
X-(Gly-Ala-Ser-Val)-Ile-Leu-Phe-Ser-Ser-Pro-Pro-Val-
Ile-(Leu-Leu-Ile-Ser)-Y;
 und

Seq.-ID.-Nr. 22
X-(Gly-Ser-Ser-Met)-Val-Leu-Phe-Ser-Ser-Pro-Pro-Val-
Ile-(Leu-Leu-Ile-Ser)-Y.

23. Synthetisches Polypeptid mit mindestens einer antigenen Stelle eines Prionproteins, umfassend eine Sequenz gemäß der Formel VI: 
X-(R31-R32-Trp-R33)-Trp-Leu-Gly-R34-R35-R36-Trp-R37-
(Trp-Leu-Gly-R38)-Y
(VI),
 wobei
R₃₁ und R₃₅ jeweils unabhängig voneinander entweder Ala oder Thr sind; R₃₂ und R₃₆ jeweils unabhängig voneinander ein aus Ser, Pro und Thr ausgewählter Aminosäurerest sind;
R₃₃ und R₃₇ jeweils unabhängig voneinander entweder Trp oder Arg sind;
R₃₄ und R₃₈ jeweils unabhängig voneinander ein aus Ala, Ser, Pro, und Thr ausgewählter Aminosäurerest sind; wobei ein oder mehrere Reste in Klammern vorhanden sein oder fehlen können, mit der Maßgabe, daß sie - wenn sie vorhanden sind - der Reihe nach an das restliche Peptid gebunden sind, und X und Y jeweils unabhängig voneinander fehlen können oder unabhängig voneinander ein oder mehrere zusätzliche Aminosäurereste sein können , mit der Maßgabe, daß - wenn vorhanden - weder X noch Y einen Teil einer Antigeneigenschaft des Prionproteins bereitstellen oder bilden, der im entsprechenden Sequenzabschnitt eines natürlichen Prionproteins an die Sequenz grenzt, an die X und Y gebunden sind.

24. Synthetisches Polypeptid mit mindestens einer antigenen Stelle eines Prionproteins, umfassend eine Sequenz gemäß der Formel VII. 
X-(R39-R40-Met-R41)-Val-Ala-Gly-R42-R43-R44-Met-R45-
(Val-Ala-Gly-R46)-Y
(VII),
 wobei R₃₉ und R₄₃ jeweils unabhängig voneinander entweder Ser oder Asn sind; R₄₀ und R₄₄ jeweils unabhängig voneinander ein aus Pro, Leu, und His ausgewählter Aminosäurerest sind, R₄₁ und R₄₅ jeweils unabhängig voneinander Val oder Glu sind; R₄₂ und R₄₆ jeweils unabhängig voneinander aus Val, Ala, Asp und Gly ausgewählt sind; wobei ein oder mehrere Reste in Klammern vorhanden sein oder fehlen können, mit der Maßgabe, daß sie - wenn sie vorhanden sind - der Reihe nach an das restliche Peptid gebunden sind, und X und Y jeweils unabhängig voneinander fehlen können oder unabhängig voneinander ein oder mehrere zusätzliche Aminosäurereste sein können, mit der Maßgabe, daß - wenn vorhanden - weder X noch Y einen Teil einer Antigeneigenschaft des Prionproteins bereitstellen oder bilden, der in dem entsprechenden Sequenzabschnitt eines natürlichen Prionproteins an die Sequenz grenzt, an die X und Y gebunden sind.

25. Synthetisches Polypeptid mit mindestens einer antigenen Stelle eines Prionproteins, umfassend eine Sequenz gemäß der Formel VIIIa oder VIIIb: 
X-(Asn-Phe-Val-His)-Asp-Cys-Val-Asn-Ile-Thr-R47-Lys-
R48-His-Thr-Val-R49-Thr-Thr-Thr-Lys-Gly-Glu-Asn-
Phe-Thr-Glu-(Thr-Asp-R50-Lys)-Y
(VIIIa),
 
X-(Met-Cys-R51-Thr)-Gln-Tyr-R52-R53-Glu-Ser-Gln-Ala-
Tyr-Tyr-R54-R55-Arg-(R56-R57-Ser-R58-R59)-Y
(VIIIb),
 wobei
R₄₇ entweder Ile oder Val ist;
R₄₈ und R₅₂ jeweils unabhängig voneinander entweder Gln oder Glu sind;
R₄₉ entweder Val oder Thr ist;
R₅₀ entweder Val oder Ile ist;
R₅₁ ein aus Ile, Thr und Val ausgewählter Aminosäurerest ist;
R₅₂ Gln oder Glu ist;
R₅₃ entweder Arg oder Lys ist;
R₅₄ entweder Asp oder Gln ist;
R₅₅ Gly ist oder fehlt;
R₅₆ entweder Gly oder Arg ist;
R₅₇ entweder Ala oder Ser ist;
R₅₈ Ser ist oder fehlt;
R₅₉ ein aus Ala, Thr, Met und Val ausgewählter Aminosäurerest ist;
ein oder mehrere Reste in Klammern vorhanden sein oder fehlen können, mit der Maßgabe, daß sie - wenn sie vorhanden sind - der Reihe nach an das restliche Peptid gebunden sind, und X und Y jeweils unabhängig voneinander fehlen können oder unabhängig voneinander ein oder mehrere zusätzliche Aminosäurereste sein können, mit der Maßgabe, daß - wenn vorhanden - weder X noch Y einen Teil einer Antigeneigenschaft des Prionproteins bereitstellen oder bilden, der im entsprechenden Sequenzabschnitt eines natürlichen Prionproteins an die Sequenz grenzt, an die X und Y gebunden sind.

26. Synthetisches Polypetid nach Anspruch 25, umfassend eine Sequenz , ausgewählt aus: 
Seq.-ID.-Nr. 32
X-(Asn-Phe-Val-His)-Asp-Cys-Val-Asn-Ile-Thr-Val-Lys-
Glu-His-Thr-Val-Thr-Thr-Thr-Thr-Lys-Gly-Glu-Asn-
Phe-Thr-Glu-(Thr-Asp-Ile-Lys)-Y;
 
Seq.-ID.-Nr. 33
X-(Met-Cys-Ile-Thr)-Gln-Tyr-Gln-Arg-Glu-Ser-Gln-Ala-
Tyr-Tyr-Gln-Arg-(Gly-Ala-Ser-Val)-Y;
 
Seq.-ID.-Nr. 34
X-(Asn-Phe-Val-His)-Asp-Cys-Val-Asn-Ile-Thr-Val-Lys-
Gln-His-Thr-Val-Thr-Thr-Thr-Thr-Lys-Gly-Glu-Asn-
Phe-Thr-Glu-(Thr-Asp-Ile-Lys)-Y;
 
Seq.-ID.-Nr. 35
X-(Met-Cys-Ile-Thr)-Gln-Tyr-Gln-Arg-Glu-Ser-Gln-Ala-
Tyr-Tyr-Gln-Arg-(Gly-Ala-Ser-Val)-Y;
 
Seq.-ID.-Nr. 36
X-(Asn-Phe-Val-His)-Asp-Cys-Val-Asn-Ile-Thr-Ile-Lys-
Gln-His-Thr-Val-Thr-Thr-Thr-Thr-Lys-Gly-Glu-Asn-
Phe-Thr-Glu-(Thr-Asp-Val-Lys)-Y;
 und

Seq.-ID.-Nr. 37
X-(Met-Cys-Ile-Thr)-Gln-Tyr-Glu-Arg-Glu-Ser-Gln-Ala-
Tyr-Tyr-Gln-Arg-(Gly-Ser-Ser-Met)-Y.

27. Subfragment eines synthetischen Polypeptides nach Anspruch 25, ausgewählt aus 
Seq.-ID.-Nr. 50
Val-Asn-Ile-Thr-Val-Lys-Gln-His-Thr-Val-Thr-Thr-Thr-Thr-
Lys-Gly-Glu-Asn-Phe-Thr-Glu-Gly-Cys;
 und

Seq.-ID.-Nr. 48
Cys-Ile-Thr-Gln-Tyr-Gln-Arg-Glu-Ser-Gln-Ala-Tyr-Tyr-Gln-Arg.

28. Synthetisches Polypeptid der allgemeinen Formel (IX);
[Lₐ-F]ₘ-[L_{b}-G]ₙ-L_{c} (IX),
wobei F und G jeweils unabhängig voneinander ein Polypeptid oder ein Subfragment nach einer der Formeln I bis VIIIb sein können, L eine Verbindungssequenz ist, a, b und c jeweils unabhängig voneinander 0 oder 1 sind, und m und n jeweils positive Zahlen sind.

29. Synthetisches Polypeptid, umfassend ein antigen signifikantes Subfragment und/oder eine antigen signifikante Variante der vorstehend identifizierten Polypeptidsequenzen nach einem der Ansprüche 1 bis 27.

30. Synthetisches Polypeptid nach einem der vorhergehenden Ansprüche, das zusätzlich ein T-Zellepitop umfaßt.

31. Synthetisches Polypeptid nach einem der vorhergehenden Ansprüche, das eine retro-inverso-Aminosäure enthält.

32. Synthetisches Polypeptid nach einem der vorhergehenden Ansprüche, das an einen Träger gebunden ist.

33. DNA-Molekül, das mindestens ein synthetisches Polypeptid nach einem der Ansprüche 1 bis 30 codiert.

34. Impfstoff, umfassend mindestens ein Polypeptid nach einem der Ansprüche 1 bis 31, das die Prophylaxe gegen Enzephalopathien wirksam fördert.

35. Kit zum Nachweisen von Prionproteinen oder Antikörpern gegen Prionproteine, das mindestens ein synthetisches Polypeptid nach einem der Ansprüche 1 bis 31 umfaßt.

36. Pharmazeutische Zusammensetzung, die als Wirkstoff mindestens ein Polypeptid oder Polypeptid-Träger-Konjugat nach einem der Ansprüche 1 bis 32 zusammen mit einem oder mehreren pharmazeutisch verträglichen Hilfsstoffen, Trägern und/oder Exzipienten enthält.

37. Verwendung eines synthetischen Polypeptides nach einem der Ansprüche 1 bis 32 für die Herstellung eines Medikamentes für die therapeutische oder prophylaktische Behandlung von Säugerenzephalopathien und/oder die Blockierung der zellulären Bindung oder Aggregation der Prionproteine.

38. Verwendung eines Polypeptides nach einem der Ansprüche 1 bis 32, entweder allein oder in Kombination mit anderen Mitteln, für die Herstellung einer Zusammensetzung zur Therapie oder Prophylaxe von Säugerenzephalopathien und/oder Stimulierung des Säuger-Immunsystems und/oder Blockierung der zellulären Bindung oder Aggregation der Prionproteine.

39. Verfahren zum Nachweis von Prionprotein oderAntikörpern gegen Prionprotein oder antigenbindenen Fragmenten davon, umfassend das Inkubieren einer Probe mit mindestens einem Polypeptid nach einem der Ansprüche 1 bis 32.

40. Verfahren zur Unterscheidung zwischen PrP^{C} und PrP^{SC}, wobei eine Probe mit einer Substanz in Kontakt gebracht wird, die aus Peptidsequenzen nach einem der Ansprüche 1 bis 32, vorzugsweise aus solchen, die die Bereiche A, B und C betreffen, und signifikanten Subfragmenten davon, Antikörpern, die gegen diese Sequenzen und Subfragmente gezüchtet wurden, ausgewählt ist, und das Vorhandensein oder das Fehlen von PrP^{SC} ermittelt wird.

41. Antikörper oder antigenbindendes Fragment davon, der/das spezifisch an ein synthetisches Polypeptid nach einem der Ansprüche 1 bis 31 bindet.

42. Kit zum Nachweisen von Prionproteinen oder Antikörpern gegen Prionproteine, das einen Antikörper oder ein antigenbindendes Fragment davon nach Anspruch 41 enthält.

43. Pharmazeutische Zusammensetzung, die als Wirkstoff einen Antikörper oder ein antigenbindendes Fragment nach Anspruch 41 zusammen mit einem oder mehreren pharmazeutisch verträglichen Trägern und/oder Exzipienten umfaßt.

44. Verwendung eines Antikörpers oder eines antigenbindenden Fragmentes nach Anspruch 41 für die Herstellung einer Zusammensetzung zur Therapie oder Prophylaxe von Säugerenzephalopathien.

45. Verfahren zum Nachweisen von Prionproteinen oder Antikörpern gegen Prionproteine, umfassend das Inkubieren einer Probe mit einem Antikörper oder einem antigenbindenden Fragment nach Anspruch 41.

46. Antiidiotypischer Antikörper, der gegen einen Antikörper oder ein antigenbindendes Fragment nach Anspruch 41 gezüchtet ist.

47. Verfahren zur Herstellung eines synthetischen Polypeptides mit mindestens einer antigenen Stelle eines Prionproteins, wobei das Verfahren die Schritte umfaßt: Kuppeln der Reste mit an sich bekannten chemischen, biologischen oder rekombinanten Techniken und Isolieren des Polypeptides nach einem der Ansprüche 1 bis 31.

48. Verfahren zur Herstellung eines Antikörpers, der spezifisch an ein synthetisches Polypeptid bindet, das mindestens eine antigene Stelle eines Prionproteins aufweist, wobei das Verfahren das Immunisieren eines nicht-menschlichen Säugers mit dem Polypeptid und das Isolieren des Antikörpers nach Anspruch 41 umfaßt.

## Revendications

1. Polypeptide synthétique ayant au moins un site antigénique d'une protéine prion, comprenant une séquence répondant aux formules générales Va, Vb et Vc : 
X-(Pro-Gly-Gly-R20)-Trp-Asn-Thr-Gly-Gly-Ser-Arg-Tyr-
Pro-Gly-Gln-Gly-Ser-Pro-Gly-Gly-Asn-Arg-Tyr-Pro-Pro-
Gln-Gly-(Gly-R21-R22-Trp)-Y     (Va) ;
 
X-(Gly-Gly-R21-R22-Trp)-Gly-Gln-Pro-His-Gly-Gly-Gly-
R23-Trp-(Gly-Gln-Pro-His)-Y (Vb) ;
 et 
X-(Gly-Gly-Gly-Trp)-Gly-Gln-Gly-Gly-R24-R25-His-R26-
Gln-Trp-Asn-Lys-Pro-R27-Lys-Pro-Lys-Thr-R28-R29-Lys
(-His-R30-Ala-Gly)-Y    (Vc)
 Dans lesquelles
R₂₀, R₂₁, R₂₃ et R₂₄ sont chacun indépendamment soit Gly soit sont absents;
R₂₂ est soit Gly soit Thr;
R₂₅ est soit Thr soit Ser;
R₂₆ est un résidu d'acide aminé choisi parmi Gly, Ser et Asn;
R₂₇ et R₂₈ sont chacun indépendamment soit Asn soit Ser;
R₂₉ est un résidu d'acide aminé choisi parmi Met, Leu et Phe;
R₃₀ est soit Val soit Met; un ou plusieurs résidus entre parenthèses peuvent être présents ou absents avec la clause conditionnelle que s'ils sont présents, ils sont fixés au reste du peptide dans la séquence; et X et Y peuvent chacun indépendamment être absents ou être indépendamment un ou plusieurs résidus d'acides aminés supplémentaires, pour autant que quand ils sont présents ni X ni Y ne donne ou ne fait partie d'une propriété antigénique de la protéine prion qui, dans la partie correspondante de la séquence d'une protéine prion naturelle, est contiguë à la séquence à laquelle X et Y sont fixés.

2. Polypeptide synthétique selon la revendication 1 comprenant une séquence choisie parmi 
Seq. I.D. No:23
X-(Pro-Gly-Gly-Gly)-Trp-Asn-Thr-Gly-Gly-Ser-Arg-Tyr-
Pro-Gly-Gln-Gly-Ser-Pro-Gly-Gly-Asn-Arg-Tyr-Pro-Pro-
Gln-Gly-(Gly-Gly-Gly-Trp)-Y ;
 
Seq. I.D. No:24
X-(Gly-Gly-Gly-Trp)-Gly-Gln-Pro-His-Gly-Gly-Gly-Trp-
(Gly-Gln-Pro-His)-Y ;
 
Seq. I.D. No:25
X-(Gly-Gly-Gly-Trp)-Gly-Gln-Gly-Gly-Thr-His-Gly-Gln-
Trp-Asn-Lys-Pro-Ser-Lys-Pro-Lys-Thr-Asn-Met-Lys
(-His-Val-Ala-Gly)-Y ;
 
Seq. I.D. No:26
X-(Pro-Gly-Gly-Gly)-Trp-Asn-Thr-Gly-Gly-Ser-Arg-Tyr-
Pro-Gly-Gln-Gly-Ser-Pro-Gly-Gly-Asn-Arg-Tyr-Pro-
Pro-Gln-Gly-(Gly-Gly-Gly-Trp)-Y ;
 
Seq. I.D. No:27
X-(Gly-Gly-Gly-Trp)-Gly-Gln-Pro-His-Gly-Gly-
Gly-Trp-(Gly-Gln-Pro-His)-Y ;
 
Seq. I.D. No:28
X-(Gly-Gly-Gly-Trp)-Gly-Gln-Gly-Gly-Ser-His-
Ser-Gln-Trp-Asn-Lys-Pro-Ser-Lys-Pro-Lys-Thr-
Asn-Met-Lys(-His-Val-Ala-Gly)-Y ;
 
Seq. I.D. No:29
X-(Pro-Gly-Gly-Gly)-Trp-Asn-Thr-Gly-Gly-Ser-Arg-Tyr-Pro-
Gly-Gln-Gly-Ser-Pro-Gly-Gly-Asn-Arg-Tyr-Pro-Pro-
Gln-Gly-(Gly-Gly-Gly-Trp)-Y ;
 
Seq. I.D. No:30
X-(Gly-Gly-Gly-Trp)-Gly-Gln-Pro-His-Gly-Gly-Gly-
Trp-(Gly-Gln-Pro-His)-Y ;
 et

Seq. I.D. No:31
X-(Gly-Gly-Gly-Trp)-Gly-Gln-Gly-Gly-Gly-Thr-His-Ser-
Gln-Trp-Asn-Lys-Pro-Ser-Lys-Pro-Lys-Thr-Asn-Met-Lys
(-His-Met-Ala-Gly)-Y.

3. Polypeptide synthétique selon la revendication 1 choisi parmi 
Seq. I.D. No:49
Gly-Gly-Trp-Asn-Thr-Gly-Gly-Ser-Arg-Tyr-Pro-Gly-Gln-
Gly-Ser-Pro-Gly-Gly-Asn-Arg-Tyr-Pro-
Pro-Gln-Gly-Gly-Gly-Cys
 
Seq. I.D. No:46
Gly-Gln-Pro-His-Gly-Gly-Gly-Trp-Gly-Gln-Pro-His-Gly-Gly-
Gly-Trp-Gly-Gln-Pro-His-Gly-Gly-Gly-Trp-Gly-Cys ;
 et

Seq. I.D. No:47
Gly-Gln-Gly-Gly-Ser-His-Ser-Gln-Trp-Asn-Lys-Pro-
Ser-Lys-Pro-Lys-Thr-Asn-Met-Lys-His-Val-Gly-Cys.

4. Polypeptide synthétique ayant au moins un site antigénique d'une protéine prion, comprenant une séquence répondant à la formule générale (I) : 
X-(R1-Lys-His-R2)-Ala-Gly-Ala-Ala-Ala-R3-Gly-Ala-Val-
Val-Gly-Gly-Leu-Gly-Gly-Tyr-Met-Leu-Gly-Ser-Ala-Met-
Ser-(Arg-Pro-R4-R5)-Y
(I)
 dans laquelle
R₁ est un résidu d'acide aminé choisi parmi Met, Leu et Phe;
R₂ est soit Met, soit Val;
R₃ est Ala ou est absent;
R₄ et R₅ sont indépendamment des résidus d'acides aminés choisis parmi Leu, Ile et Met; un ou plusieurs résidus entre parenthèses peuvent être présents ou absents avec la clause conditionnelle que s'ils sont présents, ils sont fixés au reste du peptide dans la séquence; et X et Y peuvent chacun indépendamment être absents ou être indépendamment un ou plusieurs résidus d'acides aminés supplémentaires, pour autant que quand ils sont présents ni X ni Y ne donne ou ne fait partie d'une propriété antigénique de la protéine prion qui, dans la partie correspondante de la séquence d'une protéine prion naturelle, est contiguë à la séquence à laquelle X et Y sont fixés.

5. Polypeptide synthétique selon la revendication 4 comprenant une séquence choisie parmi 
Seq. I.D. No:1
X-(Met-Lys-His-Val)-Ala-Gly-Ala-Ala-Ala-Ala-Gly-Ala-
Val-Val-Gly-Gly-Leu-Gly-Gly-Tyr-Met-Leu-Gly-Ser-Ala-
Met-Ser-(Arg-Pro-Leu-Ile)-Y ;
 et

Seq. I.D. No:2
X-(Met-Lys-His-Met)-Ala-Gly-Ala-Ala-Ala-Ala-Gly-Ala-
Val-Val-Gly-Gly-Leu-Gly-Gly-Tyr-Met-Leu-Gly-Ser-Ala-

Met-Ser-(Arg-Pro-Ile-Ile)-Y.

6. Polypeptide synthétique selon la revendication 4 constitué de la séquence Seq. I.D. No:51

Lys-His-Met-Ala-Gly-Ala-Ala-Ala-Ala-Gly-Ala-
Val-Val-Gly-Gly-Leu-Gly-Gly-Tyr-Met-Leu-Gly-Ser-Ala-
Met-Ser-Arg-Gly-Cys.

7. Sous-fragment important d'une séquence selon la revendication 4 choisi de préférence parmi 
i) X-(His-R2-Ala-Gly)-Ala-Ala-Ala-R3-Gly-Ala-Val-
Val-(Gly-Gly-Leu-Gly)-Y
 et ; 
ii) X-(Gly-Gly-Leu-Gly)-Gly-Tyr-Met-Leu-Gly-Ser-
Ala-Met-Ser-(Arg-Pro-R4-R5)-Y
 où R₂, R₃, R₄, R₅, X et Y sont tels que définis pour la formule I et un ou plusieurs résidus entre parenthèses peuvent être absents ou présents comme dans la formule I.

8. Sous-fragment selon la revendication 7 choisi parmi 
Seq. I.D. No:3
i)   X-(His-Val-Ala-Gly)-Ala-Ala-Ala-Ala-Gly-Ala-
          Val-Val-Gly-(Gly-Leu-Gly-Gly)-Y ;
 
Seq. I.D. No:4
ii) X-(Gly-Gly-Leu-Gly)-Gly-Tyr-Met-Leu-Gly-Ser-
Ala-Met-Ser-(Arg-Pro-Leu-Ile)-Y
 
Seq. I.D. No:5
i) X-(His-Met-Ala-Gly)-Ala-Ala-Ala-Ala-Gly-Ala-
Val-Val-Gly-(Gly-Leu-Gly-Gly)-Y ;
 et

Seq. I.D. No:6
ii) X-(Gly-Gly-Leu-Gly)-Gly-Tyr-Met-Leu-Gly-Ser-
Ala-Met-Ser-(Arg-Pro-Ile-Ile)-Y.

9. Polypeptide synthétique ayant au moins un site antigénique d'une protéine prion, comprenant une séquence répondant à la formule générale II : 
X-(Ser-Ala-Met-Ser)-Arg-Pro-R4-R5-His-Phe-Gly-R6-
Asp-R7-Glu-Asp-Arg-Tyr-Tyr-Arg-Glu-Asn-Met-R8-Arg-
(Tyr-Pro-Asn-Gln)-Y
(II)
 dans laquelle
R₄ et R₅ sont les mêmes que dans la formule I;
R₆ est soit Asn, soit Ser;
R₇ est soit Tyr, soit Trp;
R₈ est un résidu d'acide aminé choisi parmi His, Tyr et Asn;
un ou plusieurs résidus entre parenthèses peuvent être présents ou absents avec la clause conditionnelle que s'ils sont présents, ils sont fixés au reste du peptide dans la séquence; et X et Y peuvent chacun indépendamment être absents ou être indépendamment un ou plusieurs résidus d'acides aminés supplémentaires, pour autant que quand ils sont présents ni X ni Y ne donne ou ne fait partie d'une propriété antigénique de la protéine prion qui, dans la partie correspondante de la séquence d'une protéine prion naturelle, est contiguë à la séquence à laquelle X et Y sont fixés.

10. Polypeptide synthétique selon la revendication 9 choisi parmi 
Seq. I.D. No:7
X-(Ser-Ala-Met-Ser)-Arg-Pro-Leu-Ile-His-Phe-Gly-Ser-
Asp-Tyr-Glu-Asp-Arg-Tyr-Tyr-Arg-Glu-Asn-Met-His-Arg-
(Tyr-Pro-Asn-Gln)-Y;
 
Seq. I.D. No:8
X-(Ser-Ala-Met-Ser)-Arg-Pro-Leu-Ile-His-Phe-Gly-Asn-
Asp-Tyr-Glu-Asp-Arg-Tyr-Tyr-Arg-Glu-Asn-Met-Tyr-Arg-
(Tyr-Pro-Asn-Gln)-Y;
 et

Seq. I.D. No:9
X-(Ser-Ala-Met-Ser)-Arg-Pro-Ile-Ile-His-Phe-Gly-Ser-
Asp-Tyr-Glu-Asp-Arg-Tyr-Tyr-Arg-Glu-Asn-Met-His-Arg-
(Tyr-Pro-Asn-Gln)-Y.

11. Sous-fragment important d'une séquence selon la revendication 9 comprenant de préférence la séquence : 
X-(Ser-Ala-Met-Ser)-Arg-Pro-R4-R5-His-Phe-Gly-R6-
Asp-R7-Glu-Asp-Arg-Tyr-Tyr-(Arg-Glu-Asn-Met)-Y
 dans laquelle R₄ à R₇, X et Y sont tels que définis dans la formule II et un ou plusieurs résidus entre parenthèses peuvent être présents ou absents.

12. Polypeptide synthétique selon la revendication 11 choisi parmi 
Seq. I.D. No:42
Ser-Ala-Met-Ser-Arg-Pro-Leu-Ile-His-Phe-Gly-
Asn-Asp-Tyr-Glu-Asp-Arg-Tyr-Tyr-Gly-Cys;
 et

Seq. I.D. No:43
Ser-Ala-Met-Ser-Arg-Pro-Leu-Ile-His-Phe-Gly-
Ser-Asp-Tyr-Glu-Asp-Arg-Tyr-Tyr-Gly-Cys.

13. Sous-fragment selon la revendication 11 choisi parmi 
Seq. I.D. No:10
X-(Ser-Ala-Met-Ser)-Arg-Pro-Leu-Ile-His-Phe-Gly-Ser-
Asp-Tyr-Glu-Asp-Arg-Tyr-Tyr-(Arg-Glu-Asn-Met)-Y;
 
Seq. I.D. No:11
X-(Ser-Ala-Met-Ser)-Arg-Pro-Leu-Ile-His-Phe-Gly-Asn-
Asp-Tyr-Glu-Asp-Arg-Tyr-Tyr-(Arg-Glu-Asn-Met)-Y;
 et

Seq. I.D. No:12
X-(Ser-Ala-Met-Ser)-Arg-Pro-Ile-Ile-His-Phe-Gly-Ser-
Asp-Tyr-Glu-Asp-Arg-Tyr-Tyr-(Arg-Glu-Asn-Met)-Y.

14. Polypeptide synthétique ayant au moins un site antigénique d'une protéine prion, comprenant une séquence répondant à la formule générale III : 
X-(Asn-Met-R8-Arg)-Tyr-Pro-Asn-Gln-Val-Tyr-Tyr-Arg-
Pro-R9-Asp-R10-Tyr-R11-Asn-Gln-Asn-Asn-Phe-Val-His-
(Asp-Cys-Val-Asn)-Y
 dans laquelle
R₈ est un résidu d'acide aminé choisi parmi His, Tyr et Asn;
R₉ est Val ou Met;
R₁₀ est un résidu d'acide aminé choisi parmi Gln, Glu et Arg;
R₁₁ est Ser ou Asn; un ou plusieurs résidus entre parenthèses peuvent être présents ou absents avec la clause conditionnelle que s'ils sont présents, ils sont fixés au reste du peptide dans la séquence, et X et Y peuvent chacun indépendamment être absents ou être indépendamment un ou plusieurs résidus d'acides aminés supplémentaires, pour autant que quand ils sont présents ni X ni Y ne donne ou ne fait partie d'une propriété antigénique de la protéine prion qui, dans la partie correspondante de la séquence d'une protéine prion naturelle, est contiguë à la séquence à laquelle X et Y sont fixés.

15. Polypeptide synthétique selon la revendication 14 comprenant une séquence choisie parmi 
Seq. I.D. No:13
X-(Asn-Met-His-Arg)-Tyr-Pro-Asn-Gln-Val-Tyr-Tyr-Arg-
Pro-Val-Asp-Gln-Tyr-Ser-Asn-Gln-Asn-Asn-Phe-Val-His-
(Asp-Cys-Val-Asn)-Y ;
 
Seq. I.D. No:14
X-(Asn-Met-Tyr-Arg)-Tyr-Pro-Asn-Gln-Val-Tyr-Tyr-Arg-
Pro-Val-Asp-Arg-Tyr-Ser-Asn-Gln-Asn-Asn-Phe-Val-His-
(Asp-Cys-Val-Asn)-Y ;
 et

Seq. I.D. No:15
X-(Asn-Met-His-Arg)-Tyr-Pro-Asn-Gln-Val-Tyr-Tyr-Arg-
Pro-Met-Asp-Glu-Tyr-Ser-Asn-Gln-Asn-Asn-Phe-Val-His-
(Asp-Cys-Val-Asn)-Y.

16. Polypeptide synthétique selon la revendication 15 choisi parmi 
Seq. I.D. No:44
Asn-Met-Tyr-Arg-Tyr-Pro-Asn-Gln-Val-Tyr-Tyr-Arg-Pro-Val-
Asp-Arg-Tyr-Ser-Asn-Gln-Asn-Asn-Phe-Val-His-Gly-Cys ;
 et

Seq. I.D. No:45
Asn-Met-His-Arg-Tyr-Pro-Asn-Gln-Val-Tyr-Tyr-Arg-Pro-Val-
Asp-Gln-Tyr-Ser-Asn-Gln-Asn-Asn-Phe-Val-His-Gly-Cys.

17. Sous-fragment important d'une séquence selon la revendication 14 comprenant de préférence la séquence : 
X-(Arg-Tyr-Pro-Asn)-Gln-Val-Tyr-Tyr-Arg-Pro-R9-Asp-
R10-Tyr-R11-Asn-Gln-Asn-Asn-Phe-Val-His-
(Asp-Cys-Val-Asn)-Y.
 dans laquelle R₉, R₁₀, R₁₁, X et Y sont tels que définis dans la formule (III).

18. Sous-fragment selon la revendication 17 choisi parmi 
Seq. I.D. No:16
X-(Arg-Tyr-Pro-Asn)-Gln-Val-Tyr-Tyr-Arg-Pro-Val-Asp-
Gln-Tyr-Ser-Asn-Gln-Asn-Asn-Phe-Val-His-
(Asp-Cys-Val-Asn)-Y ;
 
Seq. I.D. No:17
X-(Arg-Tyr-Pro-Asn)-Gln-Val-Tyr-Tyr-Arg-Pro-Val-Asp-
Arg-Tyr-Ser-Asn-Gln-Asn-Asn-Phe-Val-His-
(Asp-Cys-Val-Asn)-Y ;
 et

Seq. I.D. No:18
X-(Arg-Tyr-Pro-Asn)-Gln-Val-Tyr-Tyr-Arg-Pro-Met-Asp-
Glu-Tyr-Ser-Asn-Gln-Asn-Asn-Phe-Val-His-
(Asp-Cys-Val-Asn)-Y.

19. Polypeptide synthétique ayant au moins un site antigénique d'une protéine prion, comprenant une séquence répondant à la formule générale IV : 
X-(Tyr-Tyr-R12-R13-Arg)-R14-R15-Ser-R16-R17-R18-Leu-Phe-Ser-
Ser-Pro-Pro-Val-Ile-Leu-Leu-Ile-Ser-Phe-Leu-Ile-Phe-
Leu-R19-Val-Gly-Y
(IV)
 dans laquelle
R₁₂ est Asp ou Gln;
R₁₃ est Gly ou est absent;
R₁₄ est Gly ou Arg;
R₁₅ est Ala ou Ser;
R₁₆ est Ser ou est absent;
R₁₇ est un résidu d'acide aminé choisi parmi Ala, Thr, Met et Val;
R₁₈ est Val ou Ile;
R₁₉ est Ile ou Met; un ou plusieurs résidus entre parenthèses peuvent être présents ou absents avec la clause conditionnelle que s'ils sont présents, ils sont fixés au reste du peptide dans la séquence, et X et Y peuvent chacun indépendamment être absents ou être indépendamment un ou plusieurs résidus d'acides aminés supplémentaires, pour autant que quand ils sont présents ni X ni Y ne donne ou ne fait partie d'une propriété antigénique de la protéine prion qui, dans la partie correspondante de la séquence d'une protéine prion naturelle, est contiguë à la séquence à laquelle X et Y sont fixés.

20. Polypeptide synthétique selon la revendication 19 comprenant une séquence choisie parmi 
Seq. I.D. No:19
X-(Tyr-Tyr-Gln-Arg)-Gly-Ala-Ser-Val-Ile-Leu-Phe-Ser-
Ser-Pro-Pro-Val-Ile-Leu-Leu-Ile-Ser-Phe-Leu-Ile-Phe-
Leu-Ile-Val-Gly-Y ;
 et

Seq. I.D. No:20
X-(Tyr-Tyr-Gln-Arg)-Gly-Ser-Ser-Met-Val-Leu-Phe-Ser-Ser-
Pro-Pro-Val-Ile-Leu-Leu-Ile-Ser-Phe-Leu-Ile-
Phe-Leu-Ile-Val-Gly-Y.

21. Sous-fragment important d'une séquence selon la revendication 19 comprenant de préférence la séquence : 
X-(R14-R15-Ser-R16-R17)-R18-Leu-Phe-Ser-Ser-Pro-Pro-Val-
Ile-(Leu-Leu-Ile-Ser)-Y
 Dans laquelle R₁₄ à R₁₈, X et Y sont tels que définis dans la formule IV et un ou plusieurs résidus entre parenthèses peuvent être présents ou absents comme dans la formule IV.

22. Sous-fragment selon la revendication 21 choisi parmi 
Seq. I.D. No:21
X-(Gly-Ala-Ser-Val)-Ile-Leu-Phe-Ser-Ser-Pro-Pro-Val-
Ile-(Leu-Leu-Ile-Ser)-Y ;
 et

Seq. I.D. No:22
X-(Gly-Ser-Ser-Met)-Val-Leu-Phe-Ser-Ser-Pro-Pro-Val-
Ile-(Leu-Leu-Ile-Ser)-Y.

23. Polypeptide synthétique ayant au moins un site antigénique d'une protéine prion, comprenant une séquence répondant à la formule VI : 
X-(R31-R32-Trp-R33)-Trp-Leu-Gly-R34-R35-R36-Trp-R37
(Trp-Leu-Gly-R38)-Y
(VI)
 Dans laquelle R₃₁ et R₃₅ sont chacun indépendamment soit Ala soit Thr; R₃₂ et R₃₆ sont chacun indépendamment un résidu d'acide aminé choisi parmi Ser, Pro et Thr; R₃₃ et R₃₇ sont chacun indépendamment soit Trp soit Arg; R₃₄ et R₃₈ sont chacun indépendamment un résidu d'acide aminé choisi parmi Ala, Ser, Pro et Thr; un ou plusieurs résidus entre parenthèses peuvent être présents ou absents avec la clause conditionnelle que s'ils sont présents, ils sont fixés au reste du peptide dans la séquence; et X et Y peuvent chacun indépendamment être absents ou être indépendamment un ou plusieurs résidus d'acides aminés supplémentaires, pour autant que quand ils sont présents ni X ni Y ne donne ou ne fait partie d'une propriété antigénique de la protéine prion qui, dans la partie correspondante de la séquence d'une protéine prion naturelle, est contiguë à la séquence à laquelle X et Y sont fixés.

24. Polypeptide synthétique ayant au moins un site antigénique d'une protéineprion, comprenant une séquence répondant à la formule VII : 
X-(R39-R40-Met-R41)-Val-Ala-Gly-R42-R43-R44-Met-R45-
(Val-Ala-Gly-R46)-Y
(VII)
 Dans laquelle R₃₉ et R₄₃ sont chacun indépendamment soit Ser, soit Asn; R₄₀ et R₄₄ sont chacun indépendamment un résidu d'acide aminé choisi parmi Pro, Leu et His, R₄₁ et R₄₅ sont chacun indépendamment Val ou Glu; R₄₂ et R₄₆ sont chacun indépendamment choisis parmi Val, Ala, Asp et Gly; un ou plusieurs résidus entre parenthèses peuvent être présents ou absents avec la clause conditionnelle que s'ils sont présents, ils sont fixés au reste du peptide dans la séquence; et X et Y peuvent chacun indépendamment être absents ou être indépendamment un ou plusieurs résidus d'acides aminés supplémentaires, pour autant que quand ils sont présents ni X ni Y ne donne ou ne fait partie d'une propriété antigénique de la protéine prion qui, dans la partie correspondante de la séquence d'une protéine prion naturelle, est contiguë à la séquence à laquelle X et Y sont fixés.

25. Polypeptide synthétique ayant au moins un site antigénique d'une protéine prion, comprenant une séquence répondant à la formule générale VIIIa ou VIIIb : 
X-(Asn-Phe-Val-His)-Asp-Cys-Val-Asn-Ile-Thr-R47-Lys-
R48-His-Thr-Val-R49-Thr-Thr-Thr-Lys-Gly-Glu-Asn-
Phe-Thr-Glu-(Thr-Asp-R50-Lys)-Y
(VIIIa)
 
X-(Met-Cys-R51-Thr)-Gln-Tyr-R52-R53-Glu-Ser-Gln-Ala-
Tyr-Tyr-R54-R55-Arg-(R56-R57-Ser-R58-R59)-Y
(VIIIb)
 Dans lesquelles
R₄₇ est soit Ile soit Val;
R₄₈ et R₅₂ sont chacun indépendamment soit Gln soit Glu;
R₄₉ est soit Val soit Thr;
R₅₀ est soit Val soit Ile;
R₅₁ est un résidu d'acide aminé choisi parmi Ile, Thr et Val;
R₅₂ est Gln ou Glu;
R₅₃ est soit Arg soit Lys;
R₅₄ est soit Asp soit Gln;
R₅₅ est Gly ou est absent;
R₅₆ est soit Gly soit Arg;
R₅₇ est soit Ala soit Ser;
R₅₈ est Ser ou est absent;
R₅₉ est un résidu d'acide aminé choisi parmi Ala, Thr, Met et Val;
un ou plusieurs résidus entre parenthèses peuvent être présents ou absents avec la clause conditionnelle que s'ils sont présents, ils sont fixés au reste du peptide dans la séquence; et X et Y peuvent chacun indépendamment être absents ou être indépendamment un ou plusieurs résidus d'acides aminés supplémentaires, pour autant que quand ils sont présents ni X ni Y ne donne ou ne fait partie d'une propriété antigénique de la protéine prion qui, dans la partie correspondante de la séquence d'une protéine prion naturelle, est contiguë à la séquence à laquelle X et Y sont fixés.

26. Polypeptide synthétique selon la revendication 25 comprenant une séquence choisie parmi 
Seq. I.D. No:32
X-(Asn-Phe-Val-His)-Asp-Cys-Val-Asn-Ile-Thr-Val-Lys-
Glu-His-Thr-Val-Thr-Thr-Thr-Thr-Lys-Gly-Glu-Asn-
Phe-Thr-Glu-(Thr-Asp-Ile-Lys)-Y ;
 
Seq. I.D. No:33
X-(Met-Cys-Ile-Thr)-Gln-Tyr-Gln-Arg-Glu-Ser-Gln-Ala-
Tyr-Tyr-Gln-Arg-(Gly-Ala-Ser-Val)-Y ;
 
Seq. I.D. No:34
X-(Asn-Phe-Val-His)-Asp-Cys-Val-Asn-Ile-Thr-Val-Lys-
Gln-His-Thr-Val-Thr-Thr-Thr-Thr-Lys-Gly-Glu-Asn-
Phe-Thr-Glu-(Thr-Asp-Ile-Lys)-Y ;
 
Seq. I.D. No:35
X-(Met-Cys-Ile-Thr)-Gln-Tyr-Gln-Arg-Glu-Ser-Gln-Ala-
Tyr-Tyr-Gln-Arg-(Gly-Ala-Ser-Val)-Y ;
 
Seq. I.D. No:36
X-(Asn-Phe-Val-His)-Asp-Cys-Val-Asn-Ile-Thr-Ile-Lys-
Gln-His-Thr-Val-Thr-Thr-Thr-Thr-Lys-Gly-Glu-Asn-
Phe-Thr-Glu-(Thr-Asp-Val-Lys)-Y ;
 et

Seq. I.D. No:37
X-(Met-Cys-Ile-Thr)-Gln-Tyr-Glu-Arg-Glu-Ser-Gln-Ala-
Tyr-Tyr-Gln-Arg-(Gly-Ser-Ser-Met)-Y.

27. Sous-fragment d'un polypeptide synthétique selon la revendication 25 choisi parmi 
Seq. I.D. No:50
Val-Asn-Ile-Thr-Val-Lys-Gln-His-Thr-Val-Thr-Thr-Thr-Thr-
Lys-Gly-Glu-Asn-Phe-Thr-Glu-Gly-Cys ;
 et

Seq. I.D. No:48
Cys-Ile-Thr-Gln-Tyr-Gln-Arg-Glu-Ser-Gln-Ala-Tyr-Tyr-Gln-
Arg.

28. Polypeptide synthétique de formule générale (IX) :
[Lₐ-F]ₘ-[L_{b}-G]ₙ-L_{C} (IX)
dans laquelle F et G peuvent chacun être indépendamment un polypeptide ou un sous-fragment selon l'une quelconque des formules I à VIIIb, L est une séquence de liaison, a, b et c valent chacun indépendamment 0 ou 1 et m et n sont chacun des nombres positifs.

29. Polypeptide synthétique qui comprend un sous-fragment important sur le plan antigénique et/ou une variante important sur le plan antigénique, des séquences polypeptidiques identifiées ci-dessus telles que revendiquées dans l'une quelconque des revendications 1 à 27.

30. Polypeptide synthétique selon l'une quelconque des revendications précédentes comprenant en outre un épitope de lymphocyte T.

31. Polypeptide synthétique selon l'une quelconque des revendications précédentes comprenant un rétro-inverso aminoacide.

32. Polypeptide synthétique selon l'une quelconque des revendications précédentes lié à une molécule porteuse.

33. Molécule d'ADN codant pour au moins un polypeptide synthétique selon l'une quelconque des revendications 1 à 30.

34. Vaccin comprenant au moins un polypeptide selon l'une quelconque des revendications 1 à 31 efficace pour agir en faveur d'une prophylaxie contre les encéphalopathies.

35. Trousse de détection de protéines prions ou d'anticorps dirigés contre des protéines prions, qui comprend au moins un polypeptide synthétique selon l'une quelconque des revendications 1 à 31.

36. Composition pharmaceutique contenant, comme ingrédient actif, au moins un polypeptide ou conjugué polypeptide-molécule porteuse tel que revendiqué dans l'une quelconque des revendications 1 à 32 en association avec un ou plusieurs adjuvants, supports et/ou excipients pharmaceutiquement acceptables.

37. Utilisation d'un polypeptide synthétique selon l'une quelconque des revendications 1 à 32 pour la préparation d'un médicament destiné au traitement thérapeutique ou prophylactique d'encéphalopathies de mammifères et/ou au blocage de la liaison cellulaire ou l'agrégation des protéines prions.

38. Utilisation d'un polypeptide selon l'une quelconque des revendications 1 à 32, soit isolément soit en combinaison avec d'autres agents, pour la fabrication d'une composition destinée au traitement ou au traitement prophylactique d'encéphalopathies de mammifères et/ou à la stimulation du système immunitaire de mammifères et/ou au blocage de la liaison cellulaire ou l'agrégation des protéines prions.

39. Procédé de détection d'une protéine prion ou d'anticorps dirigés contre une protéine prion ou de fragments de liaison à l'antigène de ceux-ci, qui comprend l'incubation d'un échantillon avec au moins un polypeptide selon l'une quelconque des revendications 1 à 32.

40. Procédé permettant de faire la distinction entre PrP^{C} et PrP^{SC}, dans lequel un échantillon est mis en contact avec une substance choisie parmi les séquences peptidiques telles que revendiquées dans l'une quelconque des revendications 1 à 32, de préférence celles relatives aux régions A, B et C, et les sous-fragments importants de celles-ci, les anticorps produits contre lesdits séquences et sous-fragments, et la présence ou l'absence de PrP^{SC} est déterminée.

41. Anticorps ou fragment de liaison à l'antigène de celui-ci, qui se lie spécifiquement à un polypeptide synthétique selon l'une quelconque des revendications 1 à 31.

42. Trousse de détection de protéines prions ou d'anticorps dirigés contre des protéines prions, qui contient un anticorps ou un fragment de liaison à l'antigène de celui-ci, tel que revendiqué dans la revendication 41.

43. Composition pharmaceutique comprenant, comme ingrédient actif, un anticorps ou un fragment de liaison à l'antigène de celui-ci tel que revendiqué dans la revendication 41 en association avec un ou plusieurs supports et/ou excipients pharmaceutiquement acceptables.

44. Utilisation d'un anticorps ou d'un fragment de liaison à l'antigène de celui-ci tel que revendiqué dans la revendication 41 pour la fabrication d'une composition destinée au traitement ou au traitement prophylactique d'encéphalopathies de mammifères.

45. Procédé de détection de protéines prions ou d'anticorps dirigés contre des protéines prions, qui comprend l'incubation d'un échantillon avec un anticorps ou un fragment de liaison à l'antigène de celui-ci tel que revendiqué dans la revendication 41.

46. Anticorps anti-idiotype produit contre un anticorps ou un fragment de liaison à l'antigène de celui-ci tel que revendiqué dans la revendication 41.

47. Procédé de fabrication d'un polypeptide synthétique ayant au moins un site antigénique d'une protéine prion, ledit procédé comportant les étapes de couplage des résidus à l'aide de techniques chimiques, biologiques ou de génie génétique connues en soi, et d'isolement du polypeptide tel que défini dans l'une quelconque des revendications 1 à 31.

48. Procédé de fabrication d'un anticorps qui se lie spécifiquement à un polypeptide synthétique ayant au moins un site antigénique d'une protéine prion, ledit procédé comprenant l'immunisation d'un mammifère différent de l'homme avec ledit polypeptide et l'isolement de l'anticorps tel que défini dans la revendication 41.
